(19) Europäisches Patentamt
European Patent Office
Office européen des brevets

(11) EP 4 535 001 A1

(12) EUROPEAN PATENT APPLICATION
published in accordance with Art. 153(4) EPC

(43) Date of publication:
**09.04.2025 Bulletin 2025/15**

(21) Application number: **23815793.7**

(22) Date of filing: **17.05.2023**

(51) International Patent Classification (IPC):
***G01N 33/553*** (2006.01) ***C08G 75/0268*** (2016.01)
***G01N 21/64*** (2006.01) ***G01N 33/543*** (2006.01)

(52) Cooperative Patent Classification (CPC):
**C08G 75/0268; G01N 21/64; G01N 33/543; G01N 33/553**

(86) International application number:
**PCT/JP2023/018462**

(87) International publication number:
**WO 2023/234034 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.05.2022 JP 2022089024**

(71) Applicant: **PHC Holdings Corporation**
**Tokyo 100-8403 (JP)**

(72) Inventors:
- **KOMATSU, Yoshie**
**Ehime 791-0395 (JP)**
- **KITAWAKI, Fumihisa**
**Ehime 791-0395 (JP)**

(74) Representative: **Eisenführ Speiser**
**Patentanwälte Rechtsanwälte PartGmbB**
**Postfach 10 60 78**
**28060 Bremen (DE)**

# (54) COATED METALLIC BASE, PRODUCTION METHDO THEREFOR, COMPOSITE INCLUDING COATED METALLIC BASE, AND POLYMER FOR PRODUCING COATED METALLIC BASE

(57) A coated metallic base comprising a metallic base being a metallic nanoparticle and/or a metallic film, a polymer layer covering a surface of the metallic base, and a first hydrophilic group that inhibits nonspecific adsorption to the surface of the metallic base, the first hydrophilic group being bonded to the surface of the metallic base, and the polymer layer containing a binding site with a sulfur atom interposed therein between the polymer layer and the surface of the metallic base.

Fig.1

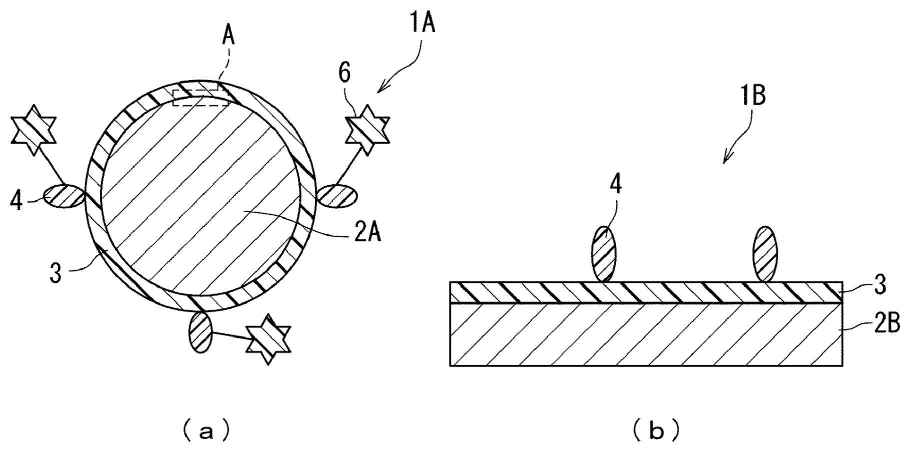


EP 4 535 001 A1

## Description

TECHNICAL FIELD

The present invention relates to a coated metallic base and a production method therefor, a composite including the coated metallic base, and a polymer for producing the coated metallic base.

BACKGROUND ART

A biosensor makes a specific test substance to be detected specifically react with a specific specifically bondable substance to form a composite, and detects the test substance by a signal caused by a specific bond in the composite.

In plasmon-excited fluorescence analysis, the composite comprises, for example, a test substance, a specifically bondable substance, a fluorescent substance, and a metallic particle. When the composite is irradiated with excitation light, surface plasmon resonance is induced in the metallic particles in the composite, and a near field in the vicinity of the surface of the metallic particle is formed. The near field increases the fluorescence intensity of the fluorescent substance.

When a particular test substance to be detected is specifically reacted with a particular specifically bondable substance to form a composite and thereby analyze the test substance, there is a problem of deterioration in signal-to-noise ratio (SN ratio) due to nonspecific adsorption to the surface of metal particles. Plasmon-excited fluorescence analysis using metal particles is also not an exception, and nonspecific adsorption to the surface of metal particles may deteriorate the SN ratio. Therefore, for example, as described in Patent Document 1, a terminal of a one-dimensional polymer (polymer chain) is bonded to the surface of metallic nanoparticles to form a polymer layer. By arranging a plurality of polymer chains on a metallic base surface so as to be substantially perpendicular to the metallic base surface, a polymer layer in which the polymer chains are arranged in a brush shape is formed.

PRIOR ART DOCUMENT

PATENT DOCUMENT

Patent Document 1: WO 2001/086301 A

SUMMARY OF THE INVENTION

PROBLEMS TO BE SOLVED BY THE INVENTION

Incidentally, the present inventors have found through intensive studies that the polymer described in Patent Document 1 has room for further improvement in detection sensitivity. Specifically, when the polymer described in Patent Document 1 is used, since a polymer layer in which one-dimensional polymer chains are arranged in a brush shape is formed, there is a possibility that the thickness of the polymer layer formed on the metallic base surface cannot be sufficiently reduced. As a result, in a composite capturing a test substance, the separative distance between the metallic bases could not be sufficiently reduced, and fluorescence could not be effectively enhanced.

In addition, since in the polymer layer, molecular chains containing hydrophobic alkyl groups are arranged in a brush shape, adsorption of a nonspecifically adsorptive substance that contributes to a noise component (nonspecific adsorption) may not be effectively inhibited.

The present invention has been devised in light of the above problems. That is, a main object of the present invention is to provide a polymer capable of reducing the thickness of a polymer layer to sufficiently reduce the separative distance between metallic bases in a composite, and improving detection sensitivity. In addition, another main object is to provide a coated metallic base including a polymer layer formed using such a polymer, a method for producing the same, and a composite including the coated metallic base.

SOLUTIONS TO THE PROBLEMS

The polymer according to one embodiment of the present invention has:
a hydrophilic group bonded with a disulfide group interposed, at the terminal of a side chain.

The coated metallic base according to one embodiment of the present invention comprises:

a metallic base being a metallic nanoparticle and/or a metallic film, a polymer layer covering a surface of the metallic base, and a first hydrophilic group that inhibits nonspecific adsorption to the surface of the metallic base,
the first hydrophilic group being bonded to the surface of the metallic base, and

the polymer layer containing a binding site with a sulfur atom interposed therein between the polymer layer and the surface of the metallic base.

The composite according to one embodiment of the present invention comprises two or more nanoparticle bodies containing the metallic nanoparticle,

wherein the two or more nanoparticle bodies include a first nanoparticle body and a second nanoparticle body, and the first nanoparticle body and the second nanoparticle body are bonded to each other with a test substance interposed therebetween.

The composite according to one embodiment of the present invention comprises a nanoparticle body containing the metallic nanoparticle and a coated metallic film containing the metallic film,

wherein the first hydrophilic group is bonded to at least one of a surface of the metallic nanoparticle and a surface of the metallic film, and
the nanoparticle body and the coated metallic film are bonded to each other with a test substance interposed therebetween.

The method for producing a coated metallic base according to one embodiment of the present invention, comprises a step of bringing the polymer into contact with a surface of a metallic base to bond the hydrophilic group to the surface of the metallic base and simultaneously form a polymer layer on the surface of the metallic base.

EFFECTS OF THE INVENTION

The present invention can provide a polymer capable of reducing the thickness of a polymer layer to sufficiently reduce the separative distance between metallic bases in a composite, and improving detection sensitivity. In addition, the present invention can provide a coated metallic base including a polymer layer formed using such a polymer, a method for producing the same, and a composite including the coated metallic base.

BRIEF DESCRIPTION OF THE DRAWINGS

Fig. 1 is a cross-sectional view schematically illustrating the coated metallic base according to the second embodiment ((a) a nanoparticle body and (b) a coated metallic film).
Fig. 2 is an enlarged schematic view of portion A in Fig. 1(a).
Fig. 3 is a cross-sectional view schematically illustrating the composite according to the third embodiment.
Fig. 4 is a diagram schematically illustrating a measuring device for detecting a test substance using the composite according to the third embodiment.
Fig. 5 is a cross-sectional view schematically illustrating the composite according to the fourth embodiment.
Fig. 6 is a diagram showing absorption spectra in the respective steps of polymer synthesis.
Fig. 7 is a diagram illustrating a reaction relating to the method for forming a polymer layer of Example 1.
Fig. 8 is an SEM image of the polymer-coated hydrophilic group-labeled silver nanoparticles of Example 1.
Fig. 9 is a diagram showing the distribution of the scattered light intensity ratio to the zeta potential for the polymer-coated hydrophilic group-labeled silver nanoparticles of Example 1.
Fig. 10 shows the distribution of the scattered light intensity ratio to the particle diameter for the polymer-coated hydrophilic group-labeled silver nanoparticles of Example 1.
Fig. 11 is a diagram illustrating an SEM image of the polymer-coated hydrophilic group-labeled silver nanoparticles of Example 2.
Fig. 12 is a diagram showing the distribution of the scattered light intensity ratio to the zeta potential for the polymer-coated hydrophilic group-labeled silver nanoparticles of Example 2.

DETAILED DESCRIPTION

Hereinafter, a nanoparticle body and a method for producing the same, a composite, and a measuring device which are embodiments of the present invention will be described in detail with reference to the embodiments illustrated in drawing. Note that the drawings include some schematic ones and may not reflect actual dimensions or proportions.
The numerical ranges referred to herein are intended to include the lower and upper limits themselves, unless

otherwise noted, such as "less than", "greater than" and "smaller than", For example, taking a numerical range such as 1 nm to 50 nm as an example, unless otherwise specified, the numerical range is interpreted as including the lower limit value "1 nm" and the upper limit value "50 nm".

In the present description, the "metallic base" refers to a base material substantially constituted of metal and includes a metallic film (or also referred to as metal plate or metal film) and a metallic nanoparticle. The metallic nanoparticle and the metallic film are different mainly in shape. In the present description, the phrase "the object member is substantially constituted of a specific material or that the object member is made of a specific material" means that the object member contains the specific material in a ratio of 95% by mass or more, 97% by mass or more, 99% by mass or more, or 100% by mass. For example, the phrase "the metallic base is substantially constituted of metal" means that the metallic base contains the metal in a ratio of 95% by mass or more, 97% by mass or more, 99% by mass or more, or 100% by mass.

In the present description, the "metallic nanoparticle" refers to a particle substantially constituted of metal, having a size on the order of nanometers (for example, several nm to 100 nm), and having a spherical or substantially spherical shape. The "metallic nanoparticle" causes localized surface plasmon resonance with other metallic nanoparticles through interaction with light (for example, visible light).

In the present description, the "metallic film" is a film substantially constituted of metal and having a thickness on the order of nanometers (for example, several nm to 100 nm). The "metallic film" causes localized surface plasmon resonance with the metallic nanoparticles through interaction with light (for example, visible light and near infrared light).

<First Embodiment: Polymer>

The polymer according to the first embodiment has a hydrophilic group bonded with a disulfide group (disulfide linkage) (-S-S-) interposed, at the terminal of a side chain.

The polymer according to the first embodiment can be brought into contact with the surface of the metallic base to form a polymer layer and hydrophilic groups on the surface of the metallic base. Thus, a coated metallic base (described in detail in the second embodiment) can be manufactured.

[Mechanism of Action]

The composite having the polymer layer formed of the polymer according to the first embodiment is superior in detection sensitivity. Without being bound by a particular theory, the reason for this is presumed as follows. The polymer according to the present embodiment has, as represented, for example, by general formula (1), a hydrophilic group bonded with a disulfide group interposed, at the terminal of a side chain,

[Chemical Formula 1]

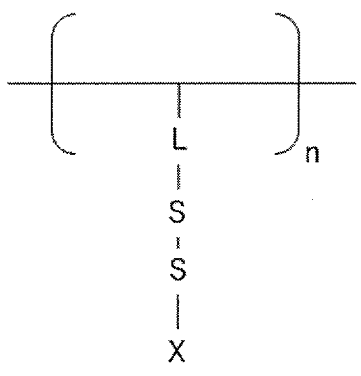

(1)

(in the general formula (1), X represents a hydrophilic group, L represents, for example, an amide linkage, and n represents the number of repeating units)

(hereinafter, the polymer represented by the general formula (1) is also referred to as "polymer (1)" in the first embodiment, and is also referred to as "polymer 3B" in the second and subsequent embodiments). The present inventors have devised that by cleaving the disulfide group, one S atom is made to contribute to reduction of the thickness of the polymer layer, and the other S atom is made to contribute to improvement of the ease of exposure of the hydrophilic group. More specifically, the present inventors have devised that the following two embodiments can

be implemented by the cleavage of a disulfide group,

(First Embodiment):
an embodiment in which one S atom attached to the terminal of a side chain branched from the polymer main chain is bonded to a prescribed position of the metallic base surface; and
(Second Embodiment):
an embodiment in which the other S atom, which is connected to a hydrophilic group X, is connected at a position different from the prescribed position on the metallic base surface.

As described above, in the present invention, it is possible to "reduce the thickness of the polymer layer" and "improve the ease of exposure of the hydrophilic group" as described above.
Specifically, when the polymer (1) is brought into contact with the metallic base, at least a part of the bond between sulfur atoms of the disulfide group is cleaved to form a binding site with a sulfur atom interposing on the surface of the metallic base. As a result, in the formed polymer layer, the polymer constituting the polymer layer is bonded to the metallic base at the terminal of the side chain. Since the polymer layer has a structure in which the main chain of the polymer constituting the polymer layer covers the metallic base surface in a two-dimensional manner such that the main chain is substantially parallel to the metallic base surface, the thickness of the polymer layer can be reduced as compared with, for example, a polymer layer formed with one-dimensional polymers arranged substantially perpendicularly to the surface of a metallic base as described in Patent Document 1. As a result, the separative distance between the metallic bases in the composite can be sufficiently reduced.
In the present description, the main chain of a polymer refers to a hydrocarbon chain (for example, an alkylene chain) that forms the main skeleton of the polymer and is parallel in a direction in which a plurality of repeating units constituting the polymer are bonded to each other and extend. This hydrocarbon chain may have a divalent group containing a hetero atom such as an ether group, an amide linkage, or an ester linkage.
In the present description, a side chain of the polymer refers to a hydrocarbon chain branched from the main chain. The hydrocarbon chain of a side chain may also have a divalent group containing a hetero atom, similarly to the hydrocarbon chain of the main chain.
In the cleavage of the bond between the sulfur atoms of the disulfide group, a hydrophilic group is bonded to the surface of the metallic base with a sulfur atom interposed in parallel with the formation of a binding site with a sulfur atom interposed therein to the surface of the metallic base. As a result, the polymer layer to be formed can have a structure in which a hydrophilic group is easily exposed. As a result, nonspecific adsorption can be inhibited, and for example, the signal-noise ratio (S/N ratio) in plasmon-excited fluorescence analysis can be improved.
For these reasons, a composite having a polymer layer formed of the polymer according to the first embodiment is superior in detection sensitivity.
In the general formula (1), n, which indicates the number of repeating units, is, for example, such a number that the weight average molecular weight Mw of the polymer (1) is larger than 12,000.

(Hydrophilic Group)

The hydrophilic group includes a polar group and/or a charged group. Examples of the polar group include at least one selected from the group consisting of a carboxyl group, a hydroxyl group, an amino group, a sulfonyl group, a phosphate group, and an alkylene oxide group. The charged group includes, for example, at least one selected from the group consisting of a group in which a polar group is charged and a zwitterionic group. The zwitterionic group includes, for example, at least one selected from the group consisting of a phosphorylcholine group and a betaine group.
The present inventors have found that since a nonspecifically adsorptive substance includes a hydrophobic site, a hydrophilic site is effective for inhibiting nonspecific adsorption to a polymer layer. Then, the present inventors have found that nonspecific adsorption can be effectively inhibited in a case where the hydrophilic site can be exposed from the polymer layer to be formed. On the basis of these technical findings, the polymer (1) having a hydrophilic group bonded with a disulfide linkage interposed, at the terminal of a side chain was derived.
The present inventors have found that introducing, in addition to a hydrophilic group, a hydrophilic site into a main chain skeleton of a polymer constituting a polymer layer is effective for inhibiting nonspecific adsorption.
From the viewpoint of further inhibiting nonspecific adsorption, the main chain skeleton of the polymer (1) preferably contains an amide linkage.
From the viewpoint of further inhibiting nonspecific adsorption, the main chain skeleton of the polymer (1) preferably has a structure derived from one or a plurality of amino acids. Such an amino acid includes, for example, at least one selected from the group consisting of lysine, histidine, arginine, glutamic acid, aspartic acid, glutamine, asparagine, serine, and threonine.
From the viewpoint of further inhibiting nonspecific adsorption, the main chain skeleton of the polymer (1)

preferably contains a biocompatible polymer as a block polymer. Examples of such a biocompatible polymer include polymethyl methacrylate.

The term "one or a plurality of" as used herein more specifically refers to "one or two or more", and "one or alternatively a plurality of" (more specifically, "one or alternatively two or more") is also synonymous.

The polymer (1) may further contain a positively charged group in a side chain. This side chain has no disulfide group. Such a positively charged group is at least one selected from the group consisting of a primary ammonium group, a secondary ammonium group, a tertiary ammonium group, a quaternary ammonium group, and a guanidyl group ($-NHC(=NH_2^+)NH_2$).

When the polymer (1) contains such a positively charged group, the polymer layer to be formed can form an electrostatic bond with the surface of the metallic base. As a result, the polymer layer is more firmly fixed.

The polymer (1) may further contain a hydrophobic group in a side chain. This side chain has no disulfide linkage. Such a hydrophobic group is at least one selected from the group consisting of an aromatic cyclic group, an aliphatic cyclic group, and an aliphatic chain group. When the polymer (1) contains such a hydrophobic group, the polymer layer to be formed can form a hydrophobic bond with the surface of the metallic base. As a result, the polymer layer is more firmly fixed.

(Method for Producing Polymer)

One example of a method for producing a polymer will be described. The method for producing a polymer includes, for example, a step of introducing a disulfide group into a side chain (disulfide group introduction step) and a step of introducing a hydrophilic group (hydrophilicity introduction step).

The method for producing the polymer (1) will be described with reference to the following Scheme 1:

[Chemical Formula 2]

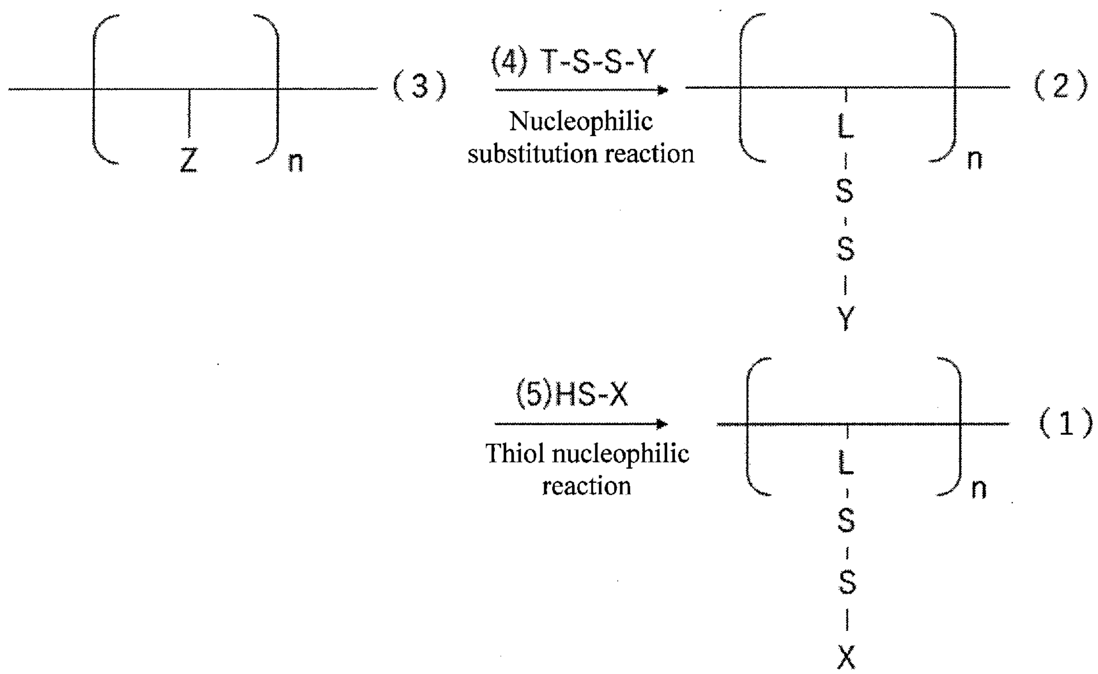


(In Scheme 1, X represents a hydrophilic group, Y represents, for example, a pyridyl group, Z and T each represent a functional site, L represents, for example, a divalent binding site, n represents the number of repeating units, and Z and T are different from each other, and bind to each other to form L.) Scheme 1 shows a reaction formula for explaining a method for producing the polymer (1). Note that L, X, and n in Scheme 1 have the same meanings as L, X, and n in general formula (1), respectively.

- Disulfide Group Introduction Step -

As shown in Scheme 1, in the disulfide group introduction step, for example, a compound having a disulfide group (compound represented by formula (4) (hereinafter, also referred to as compound (4) having a disulfide group)) is reacted

with a polymer having a functional site Z in a side chain (polymer represented by formula (3) (hereinafter, also referred to as polymer (3))) to afford a polymer having a disulfide group (polymer represented by formula (2) (hereinafter, also referred to as polymer (2))). The functional sites Z and T are different from each other and are, for example, reactive substituents such as an amino group, a carboxyl group, and an ester linkage. This reaction is, for example, a nucleophilic substitution reaction in which an amino group attacks a carboxyl group or an ester linkage to form an amide linkage. The reaction temperature is, for example, room temperature (20°C to 25°C), and the reaction time is, for example, 1 hour or more and 4 hours or less.

Examples of the functional site Z in the polymer (3) include an amino group, a carboxyl group, and an ester group. Examples of the polymer (3) include:

[Chemical Formula 3]

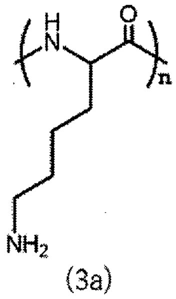
(3a)

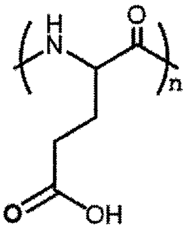
(3b)

Examples of the compound (4) having a disulfide group include:

[Chemical Formula 4]

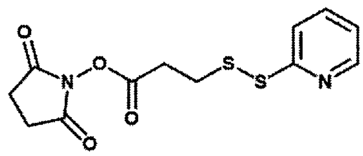
(4a)

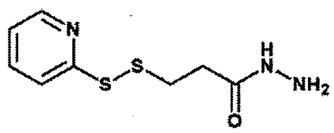
(4b)

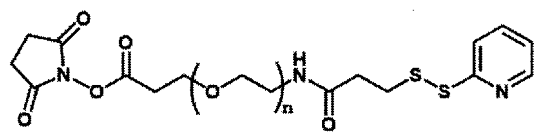
(4c)

- Hydrophilicity Introduction Step -

In the hydrophilicity introduction step, a thiol compound having a hydrophilic group (thiol compound represented by formula (5) (hereinafter, also referred to as thiol compound (5))) is reacted with the polymer (2) to afford a polymer (1) having a hydrophilic group. This reaction is a thiol nucleophilic reaction in which a thiol group attacks a disulfide linkage of the polymer (2). The reaction temperature is, for example, 35 to 40°C (particularly, °C), and the reaction time is, for example, 30 minutes or more to 2 hours or more (particularly, 1 hour or more). Examples of the thiol compound (5) include:

[Chemical Formula 5]

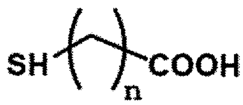

(5a)

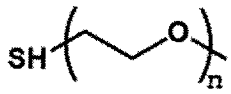

(5b)

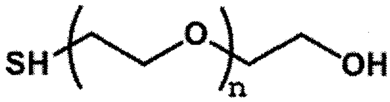

(5c)

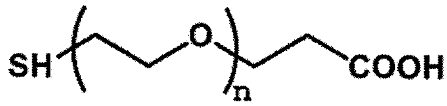

(5d)

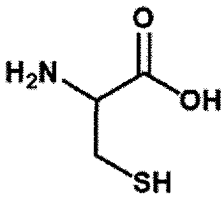

(5f)

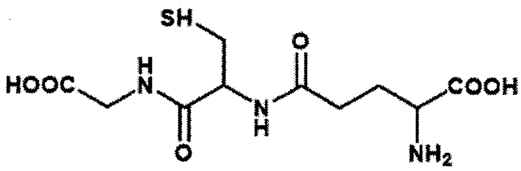

(5g)

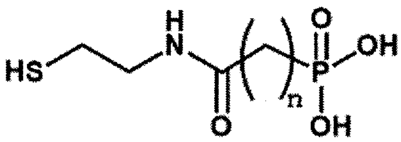

(5h)

,

and

[Chemical Formula 6]

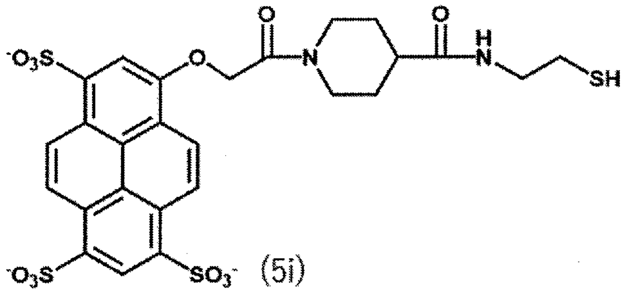

(5i)

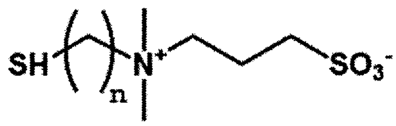

(5j)

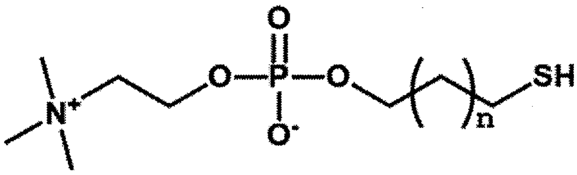

(5k)

<Second Embodiment: Coated metallic base>

The coating layer (that is, the polymer layer) of the coated metallic base according to the second embodiment can be formed of the polymer according to the first embodiment. That is, the polymer constituting the polymer layer can be derived from the polymer according to the first embodiment. In the second embodiment, the same reference numerals as those in the first embodiment have the same configurations as those in the first embodiment, and thus the description thereof will be omitted.

The coated metallic base according to the second embodiment includes a metallic base, a polymer layer covering a surface of the metallic base, and a first hydrophilic group that inhibits nonspecific adsorption to the surface of the metallic base,

the first hydrophilic group is bonded to the surface of the metallic base, and
the polymer layer contains a binding site with a sulfur atom interposed therein between the polymer layer and the surface of the metallic base.

[Mechanism of Action]

The coated metallic base according to the second embodiment can enhance detection sensitivity. Without being bound by a particular theory, the reason for this is presumed as follows. The coated metallic base according to the present embodiment contains a first hydrophilic group bonded to the surface of the metallic base. Since the first hydrophilic group inhibits nonspecific adsorption to the coated metallic base, a decrease in S/N ratio is inhibited in detection of a test substance, so that detection sensitivity is improved.

In addition, the coated metallic base according to the present embodiment includes, in addition to the first hydrophilic group, a polymer layer containing a binding site with a sulfur atom interposed therein between the polymer layer and the surface of the metallic base. As described above, since the polymer layer does not need to have a function to inhibit nonspecific adsorption, the thickness of the polymer layer can be reduced. This makes it possible to reduce the distance between the coated metallic bases in a composite to be formed in the detection of a test substance.

For these reasons, it is considered that the coated metallic base according to the second embodiment can enhance detection sensitivity.

[Basic Configuration of Coated metallic base]

The coated metallic base will be described with reference to Fig. 1. Fig. 1 is a cross-sectional view schematically illustrating the coated metallic base according to the second embodiment. As the coated metallic base 1, there are a nanoparticle body 1A and a coated metallic film 1B.

As illustrated in Fig. 1(a), a nanoparticle body 1A includes a metallic base 2 which is a metallic nanoparticle 2A, a polymer layer 3 covering a surface of the metallic base 2, and a first hydrophilic group 7A (not shown in Fig. 1(a)) that inhibits nonspecific adsorption to the surface of the metallic base 2. The nanoparticle body 1A may further include a fluorescent substance 6 and/or a specifically bondable substance 4.

The metallic nanoparticle 2A causes localized surface plasmon resonance (LSPR, hereinafter also simply referred to as "plasmon resonance") by irradiation with excitation light. The polymer layer 3 covers the surface of the metallic nanoparticles 2A, and contains a binding site with a sulfur atom interposed therein between the polymer layer 3 and the surface of the metallic base 2. The first hydrophilic group 7 is bonded to the surface of the metallic base 2.

As illustrated in Fig. 1(b), the coated metallic film 1B includes a metallic base 2 which is the metallic film 2B, a polymer layer 3 covering a surface of the metallic base 2, and a first hydrophilic group 7 (not shown in Fig. 1(b)) that inhibits nonspecific adsorption to the surface of the metallic base 2. The coated metallic film 1B may further include a specifically bondable substance 4.

The metallic film 2B causes localized surface plasmon resonance by irradiation with excitation light. The polymer layer 3 covers the surface of the metallic film 2B and includes a binding site with a sulfur atom interposed therein between the polymer layer and the surface of the metallic base 2. The first hydrophilic group 7 is bonded to the surface of the metallic base 2.

The coated metallic base 1 can be used for plasmon-excited fluorescence analysis. In other words, the coated metallic base 1 can be used for surface plasmon-field enhanced fluorescence spectroscopic immunoassay. The coated metallic base 1 can capture a test substance in a specimen and form, for example, a composite including two coated metallic bases 1 and one test substance (details will be described in the third and fourth embodiments). When the composite is irradiated with excitation light, plasmon resonance occurs to form a near field. The near field increases the fluorescence intensity. That is, an electric field enhancing effect is obtained.

The coated metallic base 1 may be blocked with a blocking agent at a nonspecific binding site. The blocked coated metallic base 1 is inhibited from forming a nonspecific bond of the specifically bondable substance 4 to a substance other than the detection target (that is, a substance other than the test substance) to reduce the background and the false positive signal, and can improve the S/N ratio). In such a case, the detection sensitivity can be further improved. Examples of the blocking agent include proteins such as bovine serum albumin (BSA), skim milk, and casein, and chemically synthesized polymers.

When the nanoparticle body 1A is present in a solvent, the dispersion of the nanoparticle body 1A may further contain a dispersant for the purpose of improving the dispersibility of the nanoparticle body 1A. Examples of such a dispersant include sodium heparin.

(Metallic base)

The metallic base 2 is covered on the surface thereof with a polymer layer 3. The metallic base 2 preferably comprises gold or silver, and more preferably comprises silver. The metallic base 2 is a metallic nanoparticle 2A and a metallic film 2B.

- Metallic Nanoparticle -

The metallic nanoparticle 2A interacts with light having a specific wavelength, which varies depending on the type of the metal constituting the metallic nanoparticle 2A, and causes localized surface plasmon resonance. In the case of silver nanoparticles (nanoparticles substantially made of silver), there is a peak of localized surface plasmon resonance at 400 nm to 530 nm, and in the case of gold nanoparticles (nanoparticles substantially made of gold), there is a peak of localized surface plasmon resonance at 510 nm to 580 nm. This varies depending on the particle diameter of the metallic nanoparticles 2A. For example, silver nanoparticles having a particle diameter of 20 nm resonate with light having a wavelength of 405 nm. Nanoparticles made of gold and having a particle diameter of 20 nm resonate with light having a wavelength of 524 nm. The particle diameter (average primary particle diameter) of the metallic nanoparticles 2 is, for example, 5 nm to 100 nm. The particle diameter of the metallic nanoparticles 2 can be determined by capturing an image of the metallic nanoparticles 2 using a scanning electron microscope (SEM) or a transmission electron microscope (TEM), measuring the particle diameter of the metallic nanoparticles 2 in the image, and calculating the average value (the number of measurements: for example, at least 10) of a plurality of particle diameters.

- Metallic Film -

The metallic film 2B interacts with light having a specific wavelength (for example, visible light), which varies depending on the type of the metal constituting the metallic film 2B, and causes localized surface plasmon resonance with metallic nanoparticles.

(Polymer Layer)

The polymer layer 3 covers the surface of the metallic base 2. The polymer layer 3 functions as a metal quenching film. In the composite, the polymer layer 3 can make a fluorescent substance 6 place apart from the surface of the metallic base 2 by at least the thickness of the polymer layer 3. Therefore, it is possible to inhibit the excited fluorescent substance 6 from coming into contact with the surface of the metallic base 2 and quenching, and to inhibit a decrease in detection sensitivity. The presence of the polymer layer 3 can be confirmed by capturing an image of the nanoparticle body 1 using SEM or TEM, and observing the coated metallic base 1 in the image.

The polymer layer 3 will be described in detail with reference to Fig. 2. Fig. 2 is an enlarged view of the portion A in Fig. 1(a), and is an enlarged schematic diagram of the vicinity of the interface between the polymer layer 3 and the surface of the metallic nanoparticle 2A in the nanoparticle body 1A. Since the polymer layer 3 of the nanoparticle body 1A has substantially the same configuration as the polymer layer 3 of the coated metallic film 1B, the polymer layer 3 of the nanoparticle body 1A will be described below.

The polymer layer 3 contains a binding site 3a with a sulfur atom interposed therein between the polymer layer 3 and the surface of the metallic nanoparticle 2 (that is, the polymer 3A constituting the polymer layer 3 contains a binding site 3a with a sulfur atom interposed therein between the polymer 3A and the surface of the metallic base 2 (metallic nanoparticle 2A)). The polymer layer 3 may further contain at least one selected from the group consisting of a positively charged group 3b and a hydrophobic group 3c (for example, the polymer 3A constituting the polymer layer 3 may further contain at least one selected from the group consisting of a positively charged group 3b and a hydrophobic group 3c in a side chain of the polymer 3A, and this side chain has no disulfide group). In this side chain, more specifically, the polymer layer 3 may further contain, in addition to the binding site 3a with a sulfur atom interposed therein, a primary ammonium group ($-NH_3^+$) as the positively charged group 3b and a hydrophobic group 3c between the polymer layer 3 and the surface of the metallic nanoparticle 2. The binding site 3a binds between the surface of the metallic nanoparticle 2 and the polymer layer 3 with a sulfur atom interposed therebetween. The positively charged group 3b forms an electrostatic bond (ionic bond) b with the surface of the negatively charged metallic nanoparticle 2. The hydrophobic group 3c forms a hydrophobic bond c with the surface of the metallic nanoparticle 2.

In the present description, a polymer constituting the polymer layer 3 is also referred to as "polymer 3A", and a polymer as a raw material for forming the polymer layer 3 via a reaction is also referred to as "polymer 3B". This is intended to make it easy to distinguish these two polymers by separating the notations of these two polymers.

All of the three bonds are relatively strong bonds with the surface of the metallic nanoparticle 2. The polymer layer 3 is stably fixed to the surface of the metallic nanoparticle 2 by the binding site 3a. Furthermore, the polymer layer 3 can be more stably fixed to the surface of the metallic nanoparticles 2 by the hydrophobic bond c and the electrostatic bond b due to the positively charged group 3b. As described above, since the polymer layer 3 is stably fixed to the surfaces of the metallic nanoparticle 2, the fluorescent substance 6 and the surface of the metallic nanoparticle 2 can be stably separated at a prescribed distance. Therefore, in the present embodiment, quenching of the excited fluorescent substance 6 is inhibited, and a decrease in detection sensitivity can be inhibited.

Furthermore, since the polymer layer 3 can comprise the polymer 3A, the polymer layer 3 is easily chemically modified as compared with a silica layer, and the necessity for surface modification or the like is low. As a result, the thickness of the polymer layer can be made smaller than that of a silica layer, and the distance between metallic nanoparticles 2A in the composite can be moderately reduced. Therefore, a near field is formed more efficiently, and the detection sensitivity can be further improved.

As illustrated in Fig. 2, the polymer 3A that can constitute the polymer layer 3 can contain, in addition to the binding site 3a with a sulfur atom interposed therein, at least one selected from the group consisting of a positively charged group 3b and a hydrophobic group 3c between the polymer 3A and the surface of the metallic nanoparticles 2. The presence of the binding site 3a with a sulfur atom interposed therein, the positively charged group 3b, and the hydrophobic group 3c can be confirmed by measuring signals derived therefrom using infrared spectroscopy, energy dispersive X-ray spectroscopy (TEM-EDS), X-ray photoelectron spectroscopy (XPS), time-of-flight secondary ion mass spectrometry (TOF-SIMS), and nuclear magnetic resonance spectroscopy.

The polymer 3A constituting the polymer layer 3 can contain, in side chains of the polymer 3A, one or a plurality of second hydrophilic groups 7B bonded with a disulfide group interposed, per molecular chain of the polymer 3A. Owing to the polymer 3A constituting the polymer layer 3 containing the second hydrophilic group 7B, nonspecific adsorption to the polymer layer 3 can be inhibited. The second hydrophilic group 7B is a hydrophilic group that is bonded to a disulfide

linkage remaining unreacted in formation of the polymer layer. The second hydrophilic group is derived, for example, from a part of hydrophilic groups in the polymer 3B (the polymer according to the first embodiment) as a raw material, the hydrophilic groups being bonded with a disulfide linkage interposed. The polymer 3A containing the second hydrophilic group 7B can be prepared by rendering the reaction conditions in the method of forming the polymer layer 3 to be mild (for example reaction conditions including a lower reaction temperature and/or a shorter reaction time). Such mild reaction conditions include, for example, a reaction time shorter than 12 to 36 hours and/or a reaction temperature lower than 20 to 30°C.

The second hydrophilic group 7B includes a polar group and/or a charged group. Examples of the polar group include at least one selected from the group consisting of a carboxyl group, a hydroxyl group, an amino group, a sulfonyl group, a phosphate group, and an alkylene oxide group. The charged group includes, for example, at least one selected from the group consisting of a group being a polar group charged and a zwitterionic group, and the zwitterionic group includes at least one selected from the group consisting of a phosphorylcholine group and a betaine group.

The polymer 3A constituting the polymer layer 3 preferably contains an amide linkage in a main chain skeleton of the polymer 3A. When the polymer 3A has an amide linkage in the main chain skeleton thereof, since the polymer 3A has hydrophilicity, nonspecific adsorption to the polymer layer 3 constituted of the polymer 3A is inhibited, so that detection sensitivity is further improved.

The polymer 3A contains a structure derived from one or a plurality of amino acids in the main chain skeleton. Such an amino acid includes, for example, at least one selected from the group consisting of lysine, histidine, arginine, glutamic acid, aspartic acid glutamine, asparagine, serine, and threonine. When the polymer 3A has a structure derived from one or a plurality of amino acids in the main chain skeleton, since the polymer 3A has hydrophilicity, nonspecific adsorption to the polymer layer 3 constituted of the polymer 3A is inhibited, so that detection sensitivity is further improved.

The polymer 3A constituting the polymer layer 3 preferably includes a biocompatible polymer as a block polymer in the main chain skeleton of the polymer 3A. When the main chain skeleton of the polymer 3A contains a biocompatible polymer as a block polymer, since the polymer 3A has hydrophilicity, nonspecific adsorption to the polymer layer 3 constituted of the polymer 3A is inhibited, so that detection sensitivity is further improved. Examples of such a biocompatible polymer include polymethacrylacrylate.

The polymer 3A constituting the polymer layer 3 can cover the surface of the metallic base 2 in a two-dimensional manner such that the main chain of the polymer 3A is substantially parallel to the surface of the metallic base 2. Such a covering mode with the polymer 3A can be realized by bonding the terminals of a plurality of side chains of the polymer 3A to the surface of the metallic base 2 with sulfur atoms interposed therebetween. In addition, since the polymer 3A of the polymer layer 3 covers the surface of the metallic base 2 such that the main chain of the polymer 3A is substantially parallel to the surface of the metallic base 2, the thickness of the polymer layer 3 can be reduced as compared with a polymer layer formed by arranging polymer chains in a brush shape (prior art: WO 2001/086301 A).

- Binding Site with Sulfur Atom Interposed Therein-

The binding site 3a with a sulfur atom interposed therein is formed, for example, by mixing a polymer having a moiety containing a disulfide linkage as a side chain with a metallic base 2 (metallic nanoparticle 2A).

- Positively Charged Group -

The positively charged group 3b can form a relatively strong electrostatic bond b with the surface of the metallic base 2 (metallic nanoparticle 2A). In the present description, the positively charged group 3b is a group having a valence of one or more and completely positively ionized. Taking into consideration a plurality of positively charged groups 3b contained in the polymer constituting the polymer layer 3, a positively charged group 3b refers to a group having a pKa of 7 or more, the pKa being represented by the following expression (1):
[Math 1]

$$pKa = pH - log\frac{[B]}{[BH^+]} \quad \cdot\cdot\cdot \quad (1)$$

in the expression (1), pKa represents a pKa of a group that is an electrically neutral group contained in the polymer 3A constituting the polymer layer 3 and can become a positively charged group (specifically, a primary ammonium group ($-NH_3^+$) or the like) 3b when positively charged (this type of group is also referred to as an electrically neutral group) (more specifically, a primary amino group ($-NH_2$) or the like); pH represents a pH of an environment where a test substance is detected (more specifically, a specimen or the like); B represents an electrically neutral group contained in the polymer 3A; and $BH^+$ represents a positively charged group 3b contained in the polymer 3A. The positively

charged group 3b refers to a group having a concentration ([$BH^+$]) of the positively charged group 3b that is 10 times or more larger than the concentration ([B]) of the electrically neutral group in the case where the electrically neutral group of the polymer 3A constituting the polymer layer 3 and the positively charged group 3b are forming an equilibrium state represented by the following chemical equilibrium formula (2)
[Chemical Formula 7]

$$B + H^+ \rightleftarrows BH^+ \cdots \qquad (2)$$

in an environment where the electrically neutral group and the positively charged group detect a test substance (for example, a specimen having a pH of about 6 to 8).

The positively charged group 3b is preferably at least one selected from the group consisting of a primary ammonium group, a secondary ammonium group, a tertiary ammonium group, a quaternary ammonium group, and a guanidyl group ($-NHC(=NH_2^+)NH_2$).

- Hydrophobic Group -

The hydrophobic group 3c can form a hydrophobic bond c with the surface of the metallic base 2 (metallic nanoparticle 2A).
The hydrophobic group 3c is, for example, at least one selected from the group consisting of an aromatic cyclic group, an aliphatic cyclic group, and an aliphatic chain group.
Examples of the aromatic cyclic group include an aromatic carbocyclic group and an aromatic heterocyclic group. The aromatic carbocyclic group is a group which does not contain any aromatic heterocyclic ring and contains an aromatic ring in which all ring atoms are carbon atoms. Examples of the aromatic carbocyclic group include aryl groups (more specifically, a phenyl group and the like) and arylalkyl groups (more specifically, a benzyl group and the like). The aromatic heterocyclic group is a group containing an aromatic ring in which at least one of the ring atoms is a hetero atom (more specifically, an oxygen atom, a sulfur atom, a nitrogen atom, or the like). Examples of the aromatic heterocyclic group include nitrogen-containing aromatic heterocyclic groups (more specifically, a pyridyl group (pyridinyl group), and the like), sulfur-containing aromatic heterocyclic groups, and oxygen-containing aromatic heterocyclic groups.
The aliphatic cyclic group is a group which does not contain any aromatic ring and contains a cyclic group composed of a non-aromatic ring. Examples of the aliphatic cyclic group include an aliphatic carbocyclic group and an aliphatic heterocyclic group. The aliphatic carbocyclic group is a group containing a non-aromatic ring in which all ring atoms are carbon atoms, and examples thereof include a cycloalkyl group. An aliphatic heterocyclic group is a group containing a non-aromatic ring in which at least one of the ring atoms is a heteroatom.
The aliphatic chain group is a chain (more specifically, linear or branched) group containing no aromatic ring and no non-aromatic ring. Examples of the aliphatic chain group include aliphatic carbon chain groups (more specifically, an alkyl group, an alkylene group, and the like) and aliphatic heterochain groups. The alkyl group is, for example, a butyl group. The alkylene group is, for example, a n-butylene group.
The thickness of the polymer layer 3 is preferably 1 nm to 50 nm, and more preferably 1 nm to 10 nm. When the thickness of the polymer layer 3 is 50 nm or less, a separative distance (separation distance) with which a near field is efficiently formed in a space between two metallic nanoparticles 2 is obtained, and thus detection sensitivity is further improved. When the thickness of the polymer layer 3 is 1 nm or more, the metallic nanoparticle 2 and a fluorescent substance are disposed at a prescribed distance, so that quenching of a fluorescent substance excited in measurement is inhibited, and detection sensitivity is further improved.
In the present description, the separative distance (separation distance) refers to the minimum value (shortest distance) of the distance between the surfaces of the metallic nanoparticles contained in two nanoparticle bodies bound with a test substance interposed therebetween in a composite.

(First Hydrophilic Group)

The first hydrophilic group 7A is bonded to the surface of the metallic base 2. More specifically, the first hydrophilic group 7A contains a binding site with a sulfur atom interposed therein between the first hydrophilic group 7A and the surface of the metallic base 2. Such a binding site can be formed, for example, by mixing the metallic base 2 (metallic nanoparticles 2A) as a starting material with the polymer 3B or a compound having a disulfide linkage (see the first embodiment).
The first hydrophilic group 7A includes a polar group and/or a charged group.
The polar group includes, for example, at least one selected from the group consisting of a carboxyl group, a hydroxyl group, an amino group, a sulfonyl group, a phosphate group, and an alkylene oxide group, and

the charged group includes, for example, at least one selected from the group consisting of a group in which the polar group is charged and a zwitterionic group, and the zwitterionic group includes at least one selected from the group consisting of a phosphorylcholine group and a betaine group.

The first hydrophilic group 7A is preferably exposed from the polymer layer 3. In such a case, nonspecific adsorption to the polymer layer 3 is further inhibited.

(Fluorescent Substance)

The fluorescent substance 6 is labeled on the surface of the metallic base 2 and/or the polymer layer 3.

From the viewpoint of inhibiting fluorescence quenching, the fluorescent substance 6 is preferably labeled on the surface of the metallic base 2 with a linker portion (for example, an alkylene group containing an amide group) interposed therebetween. When the fluorescent substance 6 is labeled on the surface of the metallic base 2 with the linker portion interposed, the linker portion is located between the fluorescent substance 6 and the surface of the metallic base 2, and an excited fluorescent substance 6 is easily prevented from coming into contact with the surface of the metallic base 2. This is considered to contribute to further inhibition of fluorescence quenching.

The fluorescent substance 6 is excited at a light wavelength at which plasmon resonance occurs, so that emits fluorescence. Examples of the fluorescent substance include complexes (metal complexes) of metals such as europium and ruthenium. Examples of a ruthenium complex include tris(bipyridine) ruthenium(II) which can have a counter anion.

The fluorescent substance 6 preferably has a large Stokes shift. Here, the Stokes shift is a difference between an absorption peak wavelength (maximum excitation wavelength) in an absorption spectrum of the fluorescent substance 6 and a fluorescence peak wavelength (maximum fluorescence wavelength) in a fluorescence spectrum of the fluorescent substance 6. When the Stokes shift of the fluorescent substance 6 is large, the absorption spectrum and the fluorescence spectrum hardly overlap each other and (scattered light of) excitation light hardly enters the fluorescence to be detected, so that more accurate fluorescence intensity can be measured.

Preferably, the fluorescence spectrum of the fluorescent substance 6 is sharp. When the fluorescence spectrum is sharp, the fluorescence spectrum hardly overlaps the absorption spectrum of the fluorescent substance 6, and thus (scattered light of) excitation light hardly enters the fluorescence to be detected, so that fluorescence intensity can be measured more accurately.

(Specifically Bondable Substance)

The specifically bondable substance 4 is a nanosized (having a size of 3 to 15 nm at the longest) substance that is to be specifically bonded to a test substance (described in detail in the third embodiment) in a specimen. The test substance is a test substance derived from a specimen, such as blood, plasma, urine, or saliva.

The specifically bondable substance 4 is at least one selected from the group consisting of an antibody (hereinafter, referred to as a nanoantibody), a ligand, an enzyme, and a nucleic acid strand (more specifically, a DNA strand and an RNA strand). In the present embodiment, the coated metallic base 1 in which such a specifically bondable substance 4 is bonded is further superior in detection sensitivity. For example, the nanoantibody as the specifically bondable substance 4 is specifically bonded to an antigen as a test substance at the tip portion (antigen binding site) of the nanoantibody through an antigen-antibody reaction to form a composite. The ligand as the specifically bondable substance 4 forms a composite by specifically protein-ligand bonding to a protein as a test substance through a ligand receptor reaction. A nucleic acid strand as the specifically bondable substance 4 forms a pair (double strand) of a nucleic acid strand and another nucleic acid strand in a complementary relationship on the basis of the complementarity of a base pair. An enzyme as the specifically bondable substance 4 forms an enzyme-substrate composite with a substrate as a test substance on the basis of the substrate specificity (stereospecificity) at the active moiety (active center) of the enzyme. These specific bonds are non-covalent bonds, for example, hydrogen bonds and bonds caused by intermolecular force, hydrophobic interaction, and charge interaction.

The nanoantibody is, for example, at least one selected from the group consisting of VHH (variable domain of heavy chain antibody) antibodies, fragmented antibodies (more specifically, Fab (fragment antigen binding) antibodies, and so on), and variants thereof. A VHH antibody is a single domain antibody. The variant is an antibody in which a part of an amino acid sequence is recombined or an antibody in which a substituent is introduced, as long as the variant has specific binding to an antigen. When the nanoantibody is at least one selected from the group consisting of VHH antibodies, fragmented antibodies, and variants thereof, because of relatively small volumes of these nanoantibodies, it is possible, for example, to reduce the distance (separation distance) between two metallic bases 2 in a composite, more efficiently form a near field, and further increase the fluorescence intensity.

The molecular mass of the nanoantibody is preferably 60,000 Da or less, more preferably 30,000 Da or less, and still more preferably 20,000 Da or less. When the molecular mass is 60,000 Da or less (in particular, 30,000 Da or less, or 20,000 Da or less), since the volume of the nanoantibody is relatively small, it is possible to reduce the separation distance

in a composite to efficiently form a near field and further increase the fluorescence intensity. Examples of the method for measuring the molecular mass include electrophoresis (SDS-PAGE), gel filtration chromatography, and static light scattering.

The specifically bondable substance 4 may be directly bonded to the polymer layer 3, or may be indirectly bonded to the polymer layer 3 with interposition of a linker portion (more specifically, $SM(PEG)_n$ wherein n is 4, 6, 8 or the like) derived from a crosslinker. Examples of such a crosslinker include an amino group-sulfhydryl group crosslinker (more specifically, an NHS-maleimide group crosslinker or the like).

[Method for Producing Coated metallic base]

The method for producing a coated metallic base 1 according to the second embodiment includes a step of bringing a polymer 3B having a hydrophilic group 7 bonded with a disulfide linkage interposed, at a terminal of a side chain into contact with a surface of a metallic base 2 to bond the hydrophilic group 7 to the surface of the metallic base 2 and simultaneously form a polymer layer 3 on the surface of the metallic base 2 (polymer layer forming step). In the polymer layer forming step, bonding of the hydrophilic group 7 to the metallic base 2 and polymer layer formation can be performed in parallel in a single step. As described above, the process can be simplified and the cost can be reduced as compared with the conventional technique. The polymer as the starting material in the method for producing a coated metallic base is the polymer according to the first embodiment.

In the reaction of bringing the polymer 3B into contact with the surface of the metallic base 2, the reaction time is, for example, 12 to 36 hours. The reaction temperature is, for example, 20 to 30°C. This reaction can be carried out under stirring conditions.

In a preferred embodiment, in the side chain, the first side chain length from the disulfide group to the hydrophilic group 7 is longer than the second side chain length from the disulfide group to the main chain of the polymer 3B. In such a case, in the polymer 3A constituting the polymer layer 3, the second side chain length corresponds to the length of the side chain bonded to the surface of the metallic base 2 (that is, the length of the side chain from the binding site with a sulfur atom interposed therein with the surface of metallic base 2 to the main chain of polymer 3 constituting the polymer layer 3). The first side chain length corresponds to a length from a binding site with a sulfur atom interposed therein with the surface of metallic base 2 to the tip of the first hydrophilic group 7A. Therefore, in such a case, the first hydrophilic group 7A is readily exposed from the polymer layer 3. Therefore, nonspecific adsorption to the polymer layer 3 can be further inhibited.

<Third Embodiment: Composite>

In the third embodiment, a composite in which two nanoparticle bodies are bound with a test substance interposed therebetween will be described as an example. In the third embodiment, the same reference numerals as those in the first and second embodiments have the same configurations as those in the first and second embodiments, respectively, and thus the description thereof will be omitted in principle.

The composite (composite nanoparticle body) according to the third embodiment can be formed by the coated metallic bases (nanoparticle bodies) according to the second embodiment capturing a test substance so as to sandwich the test substance when detecting the test substance. The composite includes two or more nanoparticle bodies.

The composite according to the third embodiment includes two or more nanoparticle bodies. The coated metallic base 1 capable of forming the composite according to the third embodiment is a nanoparticle body 1A including a metallic nanoparticle 2A as a metallic base 2, wherein a first nanoparticle body and a second nanoparticle body are included as the nanoparticle body 1A, wherein a first hydrophilic group 7A is bonded to at least one of the surface of the metallic nanoparticle 2A of the first nanoparticle body and the surface of the second metallic nanoparticle of the second nanoparticle body, and the coated metallic base 1 forms a composite in which the first nanoparticle body and the second nanoparticle body are bonded to each other with a test substance interposed therebetween.

The composite will be described with reference to Fig. 3. Fig. 3 is a cross-sectional view schematically illustrating the composite according to the third embodiment. The composite 40A according to the third embodiment includes two nanoparticle bodies 1A each containing the metallic nanoparticle 2A, the two nanoparticle bodies 1A contain a first nanoparticle body 10A and a second nanoparticle body 20A, and the first nanoparticle body 10A and the second nanoparticle body 20A are bound with a test substance 30 interposed therebetween.

Specifically, the composite 40A comprises a test substance 30 to be detected and two nanoparticle bodies 10A and 20A. In the composite 40A, the two nanoparticle bodies 10A and 20A are bound with the test substance 30 interposed therebetween. That is, the composite 40A according to the third embodiment can be formed by binding the nanoparticle bodies 1A according to the second embodiment with the test substance 30 interposed therebetween. Of the two nanoparticle bodies 10A and 20A, one is referred to as a first nanoparticle body 10A, and the other is referred to as a second nanoparticle body 20A. As described above, the composite 40A includes the first nanoparticle body 10A and the second nanoparticle body 20A as the nanoparticle body 1.

In the composite 40A, the first nanoparticle body 10A includes a first metallic nanoparticle 12A as a metallic nanoparticle, a first polymer layer 13A as a polymer layer, and a first hydrophilic group 7A (not shown in Fig. 3). Specifically, in the composite 40A, the first nanoparticle body 10A includes the first metallic nanoparticle 12A, the first polymer layer 13A covering the surface of the first metallic nanoparticle 12A, and the first hydrophilic group 7A that inhibits nonspecific adsorption to the surface of the first metallic nanoparticle 12A. The first hydrophilic group 7A is bonded to the surface of the first metallic nanoparticle 12A, and the first polymer layer 13A includes a bond with a sulfur atom interposed therein between the first polymer layer 13A and the surface of the first metallic nanoparticle 12A. The first nanoparticle body 10A further includes a first specifically bondable substance 14A bonded to the first polymer layer 13A and a first fluorescent substance 16A bonded to the first specifically bondable substance 14A.

In the composite 40A, the second nanoparticle body 20A includes a second metallic nanoparticle 22A as a metallic nanoparticle, a second polymer layer 23A as a polymer layer, and a first hydrophilic group 7A (not shown in Fig. 3). Specifically, in the composite 40A, the second nanoparticle body 20A includes the first metallic nanoparticle 22A, the second polymer layer 23A covering the surface of the second metallic nanoparticle 22A, and the first hydrophilic group 7A that inhibits nonspecific adsorption to the surface of the second metallic nanoparticle 22A. The first hydrophilic group 7A is bonded to the surface of the second metallic nanoparticle 22A, and the second polymer layer 23A includes a bond with a sulfur atom interposed therein between the second polymer layer 23A and the surface of the second metallic nanoparticle 22A. The second nanoparticle body 20A further includes a second specifically bondable substance 24A bonded to the second polymer layer 23A and a second fluorescent substance 26A bonded to the second specifically bondable substance 24A.

From the viewpoint of further enhancing the fluorescence intensity, the separation distance L is preferable to be as small as possible as long as excited fluorescent substances 16A and 26A are hardly quenched. More specifically, in a preferred embodiment, the two nanoparticle bodies 10A and 20A in the composite 40A are close to each other. In a more preferred embodiment, the two nanoparticle bodies 10A, 20A are close to each other such that the first polymer layer 13A of the first nanoparticle body 10A and the second polymer layer 23A of the second nanoparticle body 20A in the composite 40A are in contact with each other. In a still more preferred embodiment, the two nanoparticle bodies 10A and 20A are close to each other in such a manner that the first polymer layer 13A of the first nanoparticle body 10A and the second polymer layer 23A of the second nanoparticle body 20A in the composite 40A come in contact with each other with at least one of the polymer layers shrinking.

In a further preferred embodiment, in the case where the polymer layers 13A and 23A come into contact with each other with at least one of the polymer layers shrinking, for example, in the composite 40A illustrated in Fig. 3, it is considered to be possible to make at least one of the test substance 30, the specifically bondable substances 14A and 24A bonded to the test substance 30, and the fluorescent substances 16A and 26A to be embedded in the polymer layers 13A and 23A. In a still further preferred embodiment, in the case where the polymer layers 13A and 23A are in contact with each other, for example, in the composite 40A illustrated in Fig. 3, it is considered to be possible, similarly to the further preferred embodiment described above, to make at least one of the test substance 30, the specifically bondable substances 14A and 24A that are bonded to the test substance 30, and the fluorescent substances 16A and 26A to be embedded in the polymer layers 13A and 23A.

In the present embodiment, since the films covering the surfaces of the metallic nanoparticles 12A and 22A are the polymer layers 13A and 23A, the fluorescence intensity can be enhanced. The reason for this is presumed as follows. The layers covering the surfaces of the metallic nanoparticles 12A and 22A are the polymer layers 13A and 23A, and the polymer layers 13A and 23A have relatively high flexibility as compared with an inorganic film comprising an inorganic oxide. For this reason, in the composite 40A, the polymer layers 13A and 23A can shrink, so that the two metallic nanoparticles 12A and 22A can be closer to each other than a distance equivalent to a double of the thickness of each polymer layer (the thickness of the polymer layer 13A + the thickness of the polymer layer 23A). That is, since the layers covering the surfaces of the metallic nanoparticles 12A and 22A are the polymer layers 13A and 23A, the separation distance L can be less than a double of the thickness of each polymer layer. As a result, the plasmonic enhancement effect is easily obtained, and the fluorescence intensity is further enhanced. In the present description, the thickness of the polymer layer in "a double of the thickness of each polymer layer" is not the thickness of the polymer layer 13A or 23A at the shrinking portion, which serves as the target of the separation distance, but is the thickness of the polymer layer 13A or 23A at a non-shrinking portion, which does not serve as the target of the separation.

In the present embodiment, the polymer 3A constituting the polymer layers 13A and 23A contains a binding site 3a with a sulfur atom interposed therein between the surfaces of the metallic nanoparticles 12A and 22A on a side chain (more specifically, a side chain terminal) thereof. As a result, the fluorescence intensity can be further enhanced. Without being bound by a particular theory, the reason for this is presumed as follows. In such a case, it is considered that since the binding site 3a forms a bond with the surfaces of the metallic nanoparticles 12A and 22A, the polymer 3A has a network structure and covers the surfaces of the metallic nanoparticles 12A and 22A in a network manner. As described above, since the polymer 3A has a network structure, the polymer 3A further has relatively high flexibility. For this reason, in the composite 40A, the polymer layers 13A and 23A can further shrink, so that the two metallic nanoparticles 12A and 22A can

be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

In a preferred embodiment, the polymer 3A constituting the polymer layers 13A and 23A further contains, in addition to a binding site 3a with a sulfur atom interposed therein between the surfaces of the metallic nanoparticles 12A and 22A, at least one selected from the group consisting of a positively charged group 3b and a hydrophobic group 3c on a side chain (more specifically, a side chain terminal) thereof. As a result, the fluorescence intensity is further enhanced. Without being bound by a particular theory, the reason for this is presumed as follows. In such a case, since at least one selected from the group consisting of the positively charged group 3b and the hydrophobic group 3c forms a bond with the surfaces of the metallic nanoparticles 12A and 22A, it is considered that the polymer 3A has a network structure and covers the surfaces of the metallic nanoparticles 12A and 22A in a network shape. As described above, since the polymer 3A has a network structure, the polymer 3A further has relatively high flexibility. For this reason, in the composite 40A, the polymer layers 13A and 23A can further shrink, so that the two metallic nanoparticles 12A and 22A can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

The separation distance L between the first nanoparticle body 10A and the second nanoparticle body 20A is, for example, 12 nm to 52 nm, and preferably 12 nm to 27 nm. The separation distance L is a distance between the first metallic nanoparticle 12A and the second metallic nanoparticle 22A, and is a distance with which a line segment connecting between a first point P1 on the surface of the first nanoparticle body 10A and a second point P2 on the surface of the second nanoparticle body 20A is minimized. In a case where the separation distance L is 52 nm or less, when the composite 40A is irradiated with excitation light, a near field occurs more efficiently in a space near the surfaces between the first and second metallic nanoparticles 12A and 22A, so that the fluorescence intensity can be further enhanced.

The layers covering the surfaces of the metallic nanoparticles 12A and 22A are the polymer layers 13A and 23A, and the polymer layers 13A and 23A contain a binding site 3a with a sulfur atom interposed therein between the polymer layers and the surfaces of the metallic nanoparticles 12A and 22A. Therefore, as described above, the separation distance L can be smaller than the distance equivalent to a double of the thickness of each polymer layer covering the surfaces of the two metallic nanoparticles 12A and 22A in the composite 40A. For example, when the thicknesses of the polymer layers 13A and 23A are 5 nm, the separation distance L can be less than 10 nm (more specifically, 2 to 9 nm, 3 to 8 nm, 4 to 7 nm, or the like).

(Fluorescent Substance)

Preferably, as shown in Fig. 3, the fluorescent substances 16A and 26A are at least positioned between the first metallic nanoparticle 12A and the second metallic nanoparticle 22A in the composite 40A. This is because, since the near field is efficiently generated a space between the metallic nanoparticles 12A and 22A, the fluorescence intensity is easily enhanced by positioning the fluorescent substances 16A and 26A in the space between the metallic nanoparticles 12A and 22A.

(Test Substance)

The test substance 30 is a substance to be detected contained in a specimen. Examples of the test substance 30 include an antigen, a protein, a substrate, and a nucleic acid strand. The test substance 30 is specifically bonded to the specifically bondable substances 14A and 24A. For example, an antigen has at least two antigenic determinants and forms specific bonding with the first and second specifically bondable substances 14A and 24A at the antigenic determinants. Examples of the antigen include proteins such as c-reactive protein, myoglobin, troponin T, troponin I, and BNP, and antigen proteins of viruses such as influenza virus and RS virus. The test substance 30 is a test substance derived from a specimen such as blood, plasma, urine, or saliva. That is, examples of the specimen containing the test substance 30 include blood, plasma, serum, urine, and saliva. The specimen may further comprise a solvent and a buffer (more specifically, phosphate-buffered saline (PBS), Tris buffer, HEPES buffer, MOPS buffer, MES buffer, or the like).

(Method for Detecting Test Substance Using Composite)

An example of a method for detecting a test substance using the composite according to the third embodiment will be described.

The nanoparticle body 1A according to the second embodiment is dispersed in a specimen, and captures a test substance 30 contained in the specimen to form a composite 40A.

In surface plasmon-field enhanced fluorescence spectroscopy (SPFS), irradiation of the composite 40A with

excitation light causes localized surface plasmon resonance, so that a near field is efficiently formed near the surfaces of the metallic nanoparticles 12A and 22A (in particular, near the surfaces located between two metallic nanoparticles 12A and 22A). The near field and the dipole-dipole mechanisms efficiently excite the fluorescent substances 16A and 26A of the composite 40A and enhance fluorescence. By measuring the amount of fluorescence, the test substance in the specimen can be detected.

(Measuring Device for Detecting Test Substance Using Composite)

One example of a measurement method for detecting a test substance using the composite 40A according to the third embodiment will be described with reference to Fig. 4. Fig. 4 is a diagram schematically illustrating a measuring device for detecting a test substance using the composite according to the third embodiment. As illustrated in Fig. 4, the measuring device 100 includes an excitation light source 110, an excitation light radiation optical system 120, a reagent container 130, a light receiving optical system 140, and a light receiving element 150.

The excitation light source 110 emits excitation light 112. The excitation light source 110 is, for example, a laser. The excitation light radiation optical system 120 performs adjustment of the cross-sectional diameter like condensing of the excitation light 112, and outputs the incident excitation light 122. The excitation light radiation optical system 120 includes a lens 124 and a polarizing element (λ/2 plate) 126. The incident excitation light 122 output from the excitation light radiation optical system 120 is incident on the reagent container 130, and the measurement sample in the reagent container 130 is irradiated with the incident excitation light. The reagent container 130 is, for example, a detachable container (more specifically, a cell, a preparation, or the like) and a microchannel chip. The microchannel chip is a chip having a minute channel. When the reagent container 130 is a microchannel chip, for example, a nanoparticle body (reagent) according to the first embodiment and a specimen can be mixed and continuously supplied. Therefore, it is not necessary to prepare a measurement sample by mixing in advance, and it is possible to continuously perform measurement.

The measurement sample irradiated with the incident excitation light 122 emits fluorescence (detection light 132). The light receiving optical system 140 is arranged in a direction perpendicular to the traveling direction of the incident excitation light 122 toward the reagent container 130. The light receiving optical system 140 adjusts the cross-sectional diameter or the like of the detection light 132 emitted from the measurement sample, and can remove scattered light of the incident excitation light 122 or adjust the amount of light. The light receiving optical system 140 includes a lens 144 and an optical filter 146. The optical filter 146 includes, for example, a band pass filter and a dichroic mirror.

Fluorescence 142 having passed through light receiving optical system 140 is detected by the light receiving element 150. The light receiving element 150 includes, for example, a PD, an APD, a PMT, a CCD camera, and a spectroscope. The light receiving element 150 can measure the amount of fluorescence of a single wavelength, measure a fluorescence spectrum, and create two-dimensional planar fluorescence imaging.

<Fourth Embodiment: Composite>

The composite (metallic film with nanoparticle bodies) according to the fourth embodiment is different from the composite according to the third embodiment constituted of the same type of coated metallic base (nanoparticle body) in that the composite is constituted of different types of coated metallic bases (coated metallic film and nanoparticle body). In the fourth embodiment, the same reference numerals as those in the first to third embodiments have the same configurations as those in the first to third embodiments, and thus the description thereof will be omitted in principle.

The composite according to the fourth embodiment can be formed by the coated metallic bases (a coated metallic film and two or more nanoparticle bodies) according to the second embodiment capturing a test substance so as to sandwich the test substance when detecting the test substance.

The composite according to the fourth embodiment includes a coated metallic film 1B and (one or more) nanoparticle bodies 1A. The coated metallic base 1 capable of forming the composite according to the fourth embodiment includes a nanoparticle body 1A containing a metallic nanoparticle 2A as the metallic base 2 and a coated metallic film 1B containing a metallic film 2B as the metallic base 2, and a first hydrophilic group 7A is bonded to at least one of the surface of the metallic nanoparticle 2A and the surface of the metallic film 2B, and the coated metallic base 1 forms a composite in which the coated metallic film 1B and the nanoparticle body 1A are bonded to each other with a test substance interposed therebetween.

The composite includes the nanoparticle body 1A according to the second embodiment illustrated in Fig. 1(a) and the coated metallic film 1B according to the third embodiment illustrated in Fig. 1(b), and the nanoparticle body 1A and the coated metallic film 1B are bonded to each other with a test substance 30 interposed therebetween. The first hydrophilic group 7A is bonded to at least one of the surface of the metallic nanoparticle 2A and the surface of the metallic film 2B.

The composite will be described with reference to Fig. 5. Fig. 5 is a cross-sectional view schematically illustrating the composite according to the fourth embodiment. Specifically, the composite 40B includes a test substance 30 to be

detected, two or more third nanoparticle bodies 10B, and one first coated metallic base 20B. In the composite 40B, the third nanoparticle body 10B and the first coated metallic base 20B are bound with the test substance 30 interposed therebetween. That is, the composite 40B according to the fourth embodiment can be formed by binding the nanoparticle body 1A and the coated metallic film 1B according to the second embodiment with the test substance 30 interposed.

In the composite 40B, the third nanoparticle body 10B includes a third metallic nanoparticles 12B as a metallic nanoparticle, a third polymer layer 13B as a polymer layer, and a first hydrophilic group 7A (not shown in Fig. 5). Specifically, in the composite 40B, the third nanoparticle body 10B includes the third metallic nanoparticle 12B, the third polymer layer 13B covering the surface of the third metallic nanoparticle 12B, and the first hydrophilic group 7A (not shown in Fig. 5) that inhibits nonspecific adsorption to the surface of the third metallic nanoparticle 12B. The first hydrophilic group 7A is bonded to the surface of the third metallic nanoparticle 12B, and the third polymer layer 13B includes a bond with a sulfur atom interposed therein between the third polymer layer 13B and the surface of the third metallic nanoparticle 12B. The third nanoparticle body 10B further includes a third specifically bondable substance 14B bonded to the third polymer layer 13B and a third fluorescent substance 16B bonded to the third specifically bondable substance 14B.

In the composite 40B, the first coated metallic base 20B includes a first metallic film 22B as a metallic base, a fourth polymer layer 23B as a polymer layer, and a first hydrophilic group 7A (not shown in Fig. 5). Specifically, in the composite 40B, the first coated metallic base 20B includes the first metallic film 22B, the fourth polymer layer 23B covering the surface of the first metallic film 22B, and the first hydrophilic group 7A (not shown in Fig. 5) that inhibits nonspecific adsorption to the surface of the first metallic film 22B. The first hydrophilic group 7A is bonded to the surface of the first metallic film 22B, and the fourth polymer layer 23B includes a bond with a sulfur atom interposed therein between the fourth polymer layer 23B and the surface of the first metallic film 22B. The first coated metallic base 20B further includes a fourth specifically bondable substance 24B bonded to the fourth polymer layer 23B.

(Method for Detecting Test Substance Using Composite)

One example of a method for detecting a test substance using the composite according to the fourth embodiment will be described.

A measuring device illustrated in Fig. 4 is used. The first coated metallic base 20B is placed in a reagent container 130 with the fourth polymer layer 23B being exposed so that the fourth polymer layer will come into contact with the test substance 30 and the third nanoparticle body 10B described later. A dispersion containing the test substance 30 is flowed into the reagent container 130 and brought into contact with the fourth polymer layer 23B. As a result, the test substance 30 is specifically bonded to the fourth specifically bondable substance 24B bonded to the fourth polymer layer 23B. Next, a dispersion containing the third nanoparticle body 10B is made to flow into the reagent container 130 and is brought into contact with the fourth polymer layer 23B. Thus, the third nanoparticle body 10B is specifically bonded to the test substance 30 bonded to the fourth specifically bondable substance 24B of the first coated metallic base 20B. As a result, a composite 40B is formed.

Fluorescence is measured in the same manner as the measurement method in the third embodiment. Thus, the test substance is detected.

The present invention is not limited to the above-described embodiments, and can be modified in design without departing from the gist of the present invention, and at least two of the first to fourth embodiments may be combined.

In the fourth embodiment, the composite (a metallic film with a nanoparticle body) 40B is formed by bonding one nanoparticle body 10B and the metallic film 22B covered with the polymer layer 23B with one test substance 30 interposed therebetween, but the present invention is not limited thereto. The composite (a metallic film with a composite nanoparticle body) may be formed, for example, by bonding one composite 40 A and the metallic film 22B covered with the polymer layer 23B with one test substance 30 interposed therebetween.

In the second embodiment, the numbers of the fluorescent substances 6 labeled on the nanoparticle body 1A and the coated metallic film 1B are three and two, respectively, as shown in Fig. 1, but the present invention is not limited thereto. The same applies to the number of specifically bondable substances 4 (Fig. 1). The same applies to the numbers of the fluorescent substances 16A and 26A and the specifically bondable substances 14A and 24A in the composite 40A according to the third embodiment (Fig. 3). Furthermore, the same applies to the numbers of the fluorescent substance 16B and the specifically bondable substances 14B and 24B in the composite 40B according to the fourth embodiment (Fig. 5).

In the second to fourth embodiments, the fluorescent substances are bonded to the specifically bondable substances, but the present invention is not limited thereto. For example, a fluorescent substance may be bonded to a polymer layer.

In the third embodiment, the light receiving optical system 140 in the measuring device 100 is disposed in a direction perpendicular to the traveling direction of the incident excitation light 122 toward the reagent container 130, but the present invention is not limited this configuration. For example, the light receiving optical system 140 may be arranged in a direction parallel to the traveling direction of the incident excitation light 122, or may be arranged in a direction having an

acute angle or an obtuse angle with respect to the traveling direction of the incident excitation light 122.

EXAMPLES

Hereinafter, the present invention will be described in more detail with reference to Examples. The present invention is not limited by the following Examples at all. In addition, unless otherwise specified, parts and % in the Examples are on mass basis.

In Examples, the concentration of a metallic nanoparticle in a dispersion may be expressed by absorbance. The absorbance was measured using an ultraviolet-visible spectrophotometer ("infinite M200 PRO" manufactured by TECAN Japan Co., Ltd.). Note that a subscript number attached to the notation OD of the absorbance indicates an absorption wavelength. For example, "$OD_{455}$ = 0.1" indicates that the absorbance at a wavelength of 455 nm is 0.1.

<Example 1>

[Preparation of Polymer]

The polymer of Example 1 was prepared as follows.

(Introduction of Disulfide Linkage to Polymer: Disulfide Group Introduction Step)

Poly-L-lysine ("3075" manufactured by Peptide Institute, Inc.; weight average molecular weight Mw > 12,000; hereinafter also referred to as "PLL") as (3a-1) a polymer having a functional site in a side chain and 3-(2-pyridyldithio) propionamide-PEG4-NHS (manufactured by Thermo Fisher SCIENTIFIC Inc., serial number "26128", "NHS-PEG4-SPDP") as (4-1) a compound having a disulfide group were stirred and mixed under the conditions of at room temperature for 4 hours using a small rotary incubator ("RT-30 mini" manufactured by TAITEC Corporation). As a result, (2-1) a polymer having a pyridyl group (pyridinyl group) on a side chain with an SPDP linker and a disulfide linkage interposed therebetween (hereinafter, also referred to as a "polymer having a disulfide group") was obtained. This synthesis reaction is a nucleophilic substitution reaction in which a primary amino group of poly-L-lysine attacks the NHS ester group of 3-(2-pyridyldithio)propionamide-PEG4-NHS as expressed by the reaction formula (r-1).

[Chemical Formula 8]

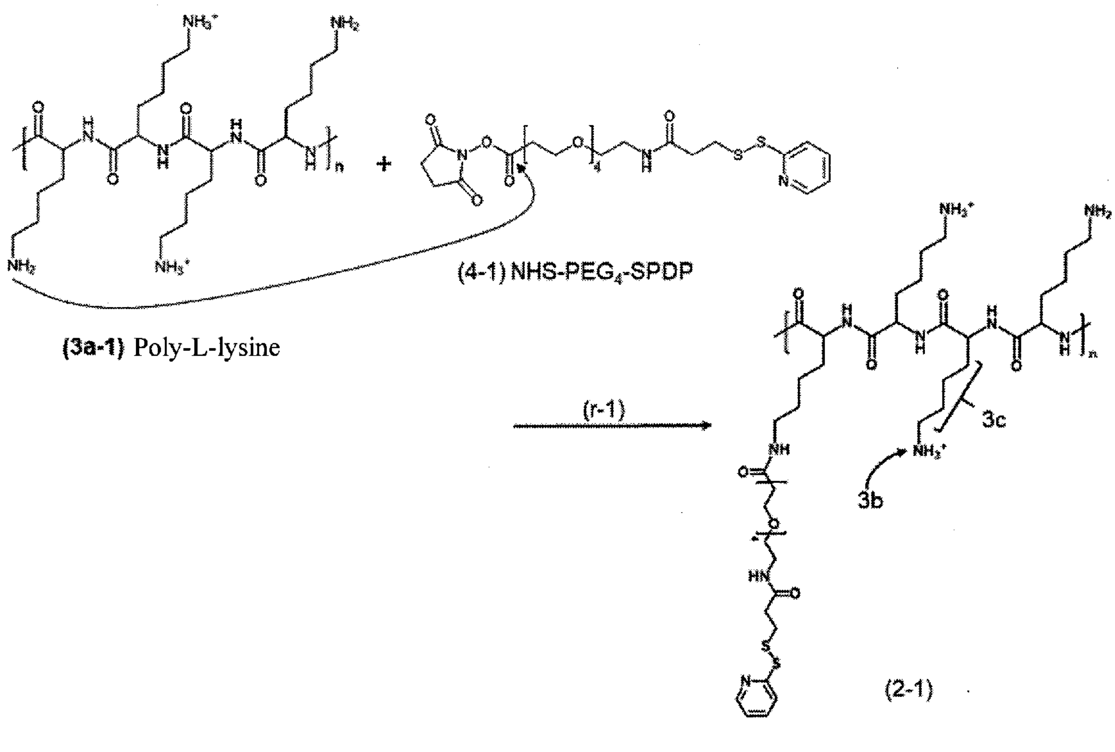


(3a-1) Poly-L-lysine

(4-1) NHS-$PEG_4$-SPDP

(2-1)

(Introduction of Hydrophilic Group to Polymer: Hydrophilic Group Introduction Step)

(2-1) A polymer having a disulfide group and (5j-1) a thiol compound having a hydrophilic group (sulfobetaine 3-undecanethiol DOJINDO LABORATORIES "S350") were stirred and mixed at 37°C and for 1 hour. As a result, (1-1) a polymer in which betaine was attached to a side chain with an SPDP linker and a disulfide linkage interposed therebetween (hereinafter, also referred to as "betaine-introduced polymer" (PLL-S-S-betaine)) was obtained. The betaine-introduced polymer (1-1) synthesized had a hydrophobic group (n-butylene group) and a positively charged group (first ammonium group). This synthesis reaction is a thiol nucleophilic reaction in which the thiol group of (5j-1) the thiol compound having a hydrophilic group attacks (2-1) the disulfide group of the polymer having a disulfide group as shown in the reaction formula (r-2).

[Chemical Formula 9]

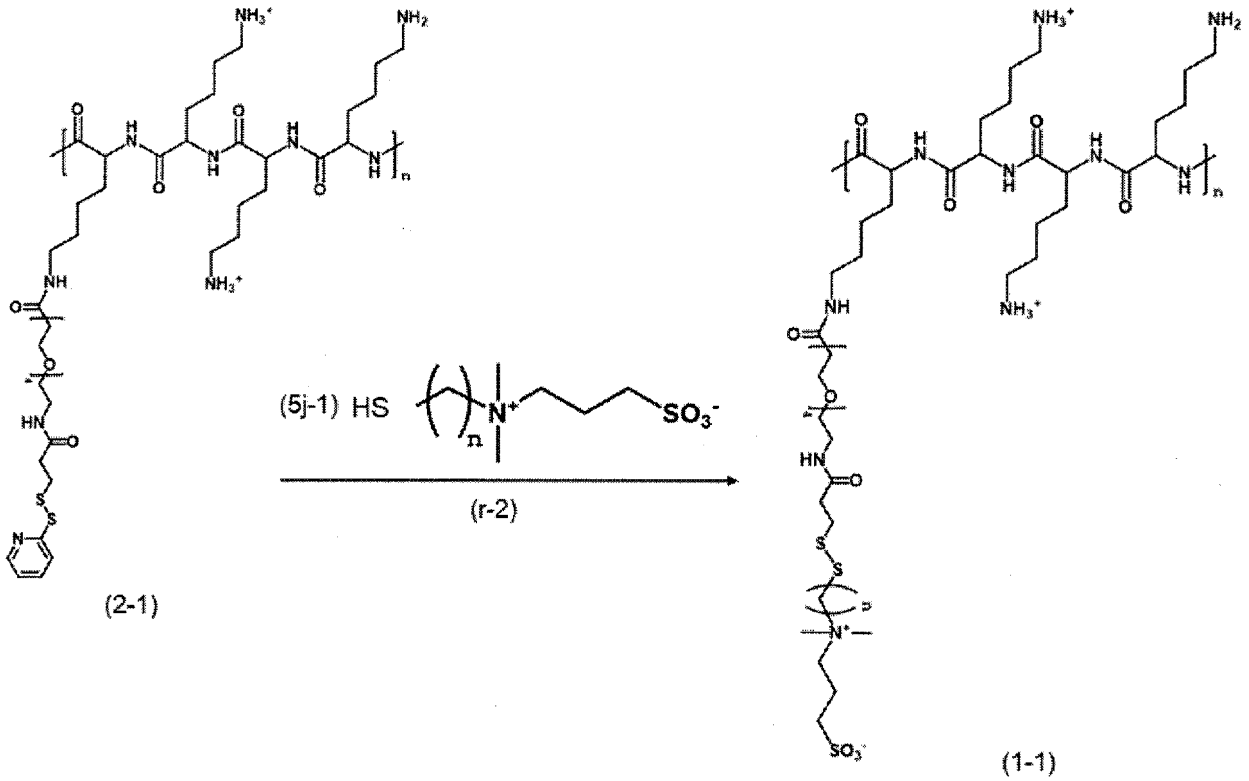


With reference to Fig. 6, the identification of (1-1) PLL-S-S-betaine will be described. Fig. 6 is a diagram showing absorption spectra in the respective steps of polymer synthesis. The solid line indicates the absorption spectrum after the reaction (r-1), the broken line indicates the absorption spectrum after the reaction (r-2), and the one-dot chain line indicates the absorption spectrum resulting from the ultrafiltration treatment after the reaction (r-2).

The absorption peak around 280 nm in the absorption spectrum (solid line) was assigned to the pyridyl group of (2-1) the polymer having a disulfide group. The absorption peak around 343 nm in the absorption spectrum (broken line) was assigned to 3-mercaptopyridine having the following resonance structure.

[Chemical Formula 10]

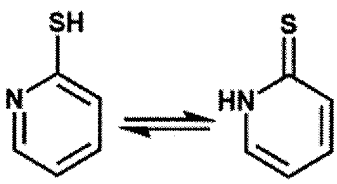

3-Mercaptopyridine is a by-product produced in the reaction (r-2). The absorption spectrum (one-dot chain line) did not have a maximum peak in the visible range. From these results, it was confirmed that the reactions (r-1) and (r-2)

proceeded and, as a result, (1-1) PPL-S-S-betaine was produced.

[Formation of Polymer Layer and Labeling of Hydrophilic Group on Surface of Metallic Nanoparticle]

The PLL-S-S-betaine (1-1) obtained was added to 1 mL of a dispersion of silver nanoparticles ("AGCB80-1M" manufactured by nanoComposix, Inc., diameter: 80 nm, $OD_{455}$ = 0.1) as the metallic nanoparticle (f), and the mixture was stirred and mixed under the conditions of at room temperature overnight using a small rotary incubator ("RT-30 mini" manufactured by TAITEC Corporation). As a result, a dispersion of silver nanoparticles covered with a polymer layer and labeled with a hydrophilic substance (betaine) (hereinafter, also referred to as "hydrophilic group-labeled polymer-coated silver nanoparticles") was obtained. In this reaction, the disulfide group of the polymer having a hydrophilic group (1-1) is cleaved by the reducing action of silver. The poly-L-lysine moiety generated by the cleavage and the hydrophilic group are each bonded to the surface of the silver nanoparticle with a sulfur atom interposed therebetween. As a result, as shown in Fig. 7 (the diagram showing the reaction relating to the method for forming the polymer layer of Example 1), betaine as a hydrophilic substance is labeled on the surface of the silver nanoparticle with a sulfur atom interposed therebetween, and the polymer layer is bonded to the surface of the silver nanoparticle with a sulfur atom interposed therebetween. That is, in this step, the labeling of the hydrophilic group and the formation of the polymer layer proceed in parallel.

[Evaluation Method]

(SEM Image)

An SEM image (300 K magnifications) of the obtained hydrophilic group-labeled polymer-coated silver nanoparticles of Example 1 was taken using a scanning electron microscope ("Regulus 8220" manufactured by Hitachi High-Tech Corporation). Fig. 8 shows an SEM image of the hydrophilic group-labeled polymer-coated silver nanoparticles of Example 1. In the SEM image obtained as shown in Fig. 8, it was confirmed that the silver nanoparticles were covered with a polymer layer.

(Measurement and Evaluation by Zeta Potential and Particle Size Distribution)

The zeta potential and the particle size distribution of the hydrophilic group-labeled polymer-coated silver nanoparticles of Example 1 were measured using a zeta potential analyzer ("ZETA SIZER Nanoseries nano-ZS" manufactured by MALVERN) (the number of measurements = 3). The measurement result of the zeta potential will be described with reference to Fig. 9. Fig. 9 is a diagram showing the distribution of the scattered light intensity ratio to the zeta potential (zeta potential distribution) for the polymer-coated hydrophilic group-labeled silver nanoparticles of Example 1 (specifically, the distribution is one of the distributions obtained by three measurements). The distribution of the scattered light intensity ratio to the zeta potential was a distribution in which the peaks were present at -5.77 to -2.33 mV. The polymer-coated hydrophilic group-labeled silver nanoparticles were slightly negatively charged overall.

The measurement result of the particle size distribution will be described with reference to Fig. 10. Fig. 10 is a diagram showing the distribution of the scattered light intensity ratio to the particle diameter (particle size distribution) for the polymer-coated hydrophilic group-labeled silver nanoparticles of Example 1 (specifically, the distribution is one of the distributions obtained by three measurements). The distribution of the scattered light intensity ratio to the particle diameter was 97.93 nm (expected value M) $\pm$ 0.163 nm (polydispersity index (PDI)).

<Example 2>

(2-1) A polymer having a disulfide group (PLL-S-S-PC) was obtained in the same manner as in Example 1 except that the thiol compound having a hydrophilic group in the reaction (r-2) was changed from (5j-1) to a thiol compound having a phosphorcholine group represented by (5k-1) ("product code; MCP-03-0001-PC" manufactured by Medicinal Chemistry Pharmaceutical, Co., Ltd.):

[Chemical Formula 11]

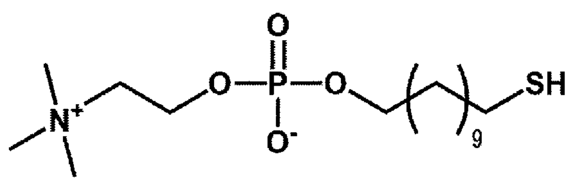

(5k-1). An absorption spectrum similar to that in Example 1 was obtained.

An SEM image (300 K magnifications) of the hydrophilic group-labeled polymer-coated silver nanoparticles of Example 2 was taken, and a zeta potential distribution and a particle size distribution were obtained. Fig. 11 shows the SEM image of the hydrophilic group-labeled polymer-coated silver nanoparticles of Example 2. It was confirmed from Fig. 11 that the silver nanoparticles were covered with a polymer layer. The hydrophilic group-labeled polymer-coated silver nanoparticles of Example 2 were measured using time-of-flight secondary ion mass spectrometry (TOF-SIMS). As a result, the presence of phosphoric acid could be confirmed in the region where the silver nanoparticles existed. It was concluded that this phosphoric acid was a fragment derived from a phosphorcholine group bonded to the surface of a silver nanoparticle. Fig. 12 shows the zeta potential distribution for the polymer-coated hydrophilic group-labeled silver nanoparticles of Example 2 (specifically, the distribution is one of the distributions obtained by three measurements). From the zeta potential distribution, these peaks were present at +20 to +27 mV. The polymer-coated hydrophilic group-labeled silver nanoparticles were slightly positively charged overall. Table 1:

[Table 1]

| | Expected value M (nm) | Polydispersity index PDI (nm) |
|---|---|---|
| Example 1 | 97.93 | 0.163 |
| Example 2 | 91.03 | 0.085 |
| Comparative Example 1 | 86.79 | 0.067 |

shows the expected value M and the polydispersity index PDI regarding the particle size distribution of the hydrophilic group-labeled polymer-coated silver nanoparticles of Example 2.

<Comparative Example 1>

In Comparative Example 1, polymer-coated silver nanoparticles were prepared in the same manner as in Example 1 except that the polymer for covering the silver nanoparticles was changed from (1-1) PLL-S-betaine to PLL ("3075" manufactured by Peptide Institute, Inc.). In Comparative Example 1, the zeta potential distribution was measured in the same manner as in Example 1. The zeta potential distribution of Comparative Example 1 was a distribution in which the peak was present around +50 mV. The polymer-coated silver nanoparticles were positively charged overall.

<Comparative Example 2>

In Comparative Example 2, the particle size distribution and the zeta potential distribution were measured in the same manner as in Example 1 except that silver nanoparticles ("AGCB80-1M" manufactured by nanoComposix, diameter: 80 nm) were used instead of the polymer-coated hydrophilic group-labeled silver nanoparticles. The zeta potential distribution of Comparative Example 2 was a distribution in which the peak was present around -50 mV. The silver nanoparticles were negatively charged overall.

(Comparison of Examples 1 and 2 and Comparative Examples 1 and 2)

In the zeta potential distributions of Examples 1 and 2, the absolute values of the zeta potential at the peak positions were smaller than the absolute values of the zeta potential at the peak positions of Comparative Examples 1 and 2. Usually, when the zeta potential approaches around 0 mV, aggregation occurs, and it is difficult to disperse particles in a primary particle state. Nevertheless, in the results of the particle size distribution of Examples 1 and 2, the expected values were only slightly larger than the particle diameter of silver nanoparticles (80 nm). This increase is considered to be equivalent to the thickness of the polymer layer. In addition, the PDI was not so large. In consideration of these, it is considered that the hydrophilic group-labeled polymer-coated silver nanoparticles (coated metallic base) are dispersed in a primary particle state. This is considered to be due to the presence of a hydrophilic group on the surface of the metallic base. For these reasons, it is considered that the adsorption of the nonspecifically adsorptive substance contributing to the noise component can be effectively inhibited.

The embodiments of the coated metallic base, the production method therefor, the composite including the coated metallic base, and the polymer for producing the coated metallic base according to the present disclosure are as follows.

<1> A coated metallic base comprising a metallic base being a metallic nanoparticle and/or a metallic film, a polymer layer covering a surface of the metallic base, and a first hydrophilic group that inhibits nonspecific adsorption to the

surface of the metallic base,

the first hydrophilic group being bonded to the surface of the metallic base, and
the polymer layer containing a binding site with a sulfur atom interposed therein between the polymer layer and the surface of the metallic base.

<2> The coated metallic base according to <1>, wherein the first hydrophilic group includes a binding site with a sulfur atom interposed therein between the first hydrophilic group and the surface of the metallic base.
<3> The coated metallic base according to <1> or <2>, wherein a polymer forming the polymer layer contains, in a side chain of the polymer, a binding site with the sulfur atom interposed therein between the side chain and the surface of the metallic base.
<4> The coated metallic base according to any one of <1> to <3>, wherein a polymer constituting the polymer layer contains, in a side chain of the polymer, one or a plurality of second hydrophilic groups bonded with a disulfide group interposed, per one molecular chain of the polymer.
<5> The coated metallic base according to <4>,

wherein the polymer further contains the second hydrophilic group in a side chain of the polymer,
the first hydrophilic group and the second hydrophilic group each independently include a polar group and/or a charged group,
the polar group includes at least one selected from the group consisting of a carboxyl group, a hydroxyl group, an amino group, a sulfonyl group, a phosphate group, and an alkylene oxide group,
the charged group includes at least one selected from the group consisting of a group in which the polar group is charged and a zwitterionic group, and the zwitterionic group includes at least one selected from the group consisting of a phosphorylcholine group and a betaine group.

<6> The coated metallic base according to any one of <1> to <5>, wherein a polymer constituting the polymer layer contains an amide linkage in a main chain skeleton of the polymer.
<7> The coated metallic base according to any one of <1> to <6>,

wherein a polymer constituting the polymer layer further contains a positively charged group in a side chain of the polymer,
the side chain has no disulfide linkage,
the positively charged group is at least one selected from the group consisting of a primary ammonium group, a secondary ammonium group, a tertiary ammonium group, a quaternary ammonium group, and a guanidyl group $(-NHC(=NH_2^+)NH_2)$.

<8> The coated metallic base according to any one of <1> to <7>,

wherein a polymer constituting the polymer layer further contains a hydrophobic group in a side chain of the polymer,
the side chain has no disulfide linkage, and
the hydrophobic group is at least one selected from the group consisting of an aromatic cyclic group, an aliphatic cyclic group, and an aliphatic chain group.

<9> The coated metallic base according to any one of <3> to <8>,

wherein the polymer contains a structure derived from one or a plurality of amino acids in a main chain skeleton, and
the amino acids comprise at least one selected from the group consisting of lysine, histidine, arginine, glutamic acid, aspartic acid glutamine, asparagine, serine, and threonine.

<10> The coated metallic base according to any one of <1> to <9>, wherein a polymer constituting the polymer layer contains a biocompatible polymer as a block polymer in a main chain skeleton of the polymer.
<11> The coated metallic base according to any one of <1> to <10>, further comprising a fluorescent substance and/or a specifically bondable substance that is to be specifically bonded to a test substance in a specimen.
<12> The coated metallic base according to <11>, wherein the specifically bondable substance is a nanoantibody.
<13> The coated metallic base according to any one of <1> to <12>, wherein the polymer layer has a thickness of 1 nm to 10 nm.

<14> The coated metallic base according to any one of <1> to <13>, wherein the coated metallic base is a coated metallic base to be used for plasmon-excited fluorescence analysis.
<15> The coated metallic base according to any one of <1> to <14>, wherein the metallic base comprises gold or silver.
<16> The coated metallic base according to any one of <1> to <15>,

which is a nanoparticle body including the metallic nanoparticle as the metallic base,
wherein a first nanoparticle body and a second nanoparticle body are included as the nanoparticle body,
wherein the first hydrophilic group is bonded to at least one of a surface of the metallic nanoparticle of the first nanoparticle body and a surface of the metallic nanoparticle of the second nanoparticle body, and
the coated metallic base forms a composite in which the first nanoparticle body and the second nanoparticle body are bonded to each other with a test substance interposed therebetween.

<17> The coated metallic base according to any one of <1> to <16>,

wherein the coated metallic base includes a nanoparticle body containing the metallic nanoparticle as the metallic base and a coated metallic film containing the metallic film as the metallic base,
the first hydrophilic group is bonded to at least one of a surface of the metallic nanoparticle and a surface of the metallic film, and
the coated metallic base forms a composite in which the coated metallic film and the nanoparticle body are bonded to each other with a test substance interposed therebetween.

<18> The coated metallic base according to <16> or <17>, wherein the test substance is a test substance derived from a specimen being blood, plasma, urine, or saliva.
<19> The coated metallic base according to any one of <1> to <18>, wherein a molecule constituting the polymer layer cover a surface of the metallic base in a two-dimensional manner such that a main chain of the polymer is substantially parallel to the surface of the metallic base.
<20> A composite comprising two or more nanoparticle bodies containing the metallic nanoparticle according to any one of <1> to <19>,

the two or more nanoparticle bodies include a first nanoparticle body and a second nanoparticle body, and
the first nanoparticle body and the second nanoparticle body are bonded to each other with a test substance interposed therebetween.

<21> A composite comprising a nanoparticle body including the metallic nanoparticle according to any one of <1> to <19> and a coated metallic film including the metallic film according to any one of <1> to <19>,

wherein the first hydrophilic group is bonded to at least one of a surface of the metallic nanoparticle and a surface of the metallic film, and
the nanoparticle body and the coated metallic film are bonded to each other with a test substance interposed therebetween.

<22> A polymer having a hydrophilic group bonded with a disulfide group interposed, at a terminal of a side chain.
<23> The polymer according to <22>,

wherein the hydrophilic group includes a polar group and/or a charged group,
the polar group includes at least one selected from the group consisting of a carboxyl group, a hydroxyl group, an amino group, a sulfonyl group, a phosphate group, and an alkylene oxide group, and
the charged group includes at least one selected from the group consisting of a group in which the polar group is charged and a zwitterionic group, and the zwitterionic group includes at least one selected from the group consisting of a phosphorylcholine group and a betaine group.

<24> The polymer according to <22> or <23>,

wherein the polymer further has a positively charged group in a side chain of the polymer,
the side chain has no disulfide linkage, and
the positively charged group is at least one selected from the group consisting of a primary ammonium group, a secondary ammonium group, a tertiary ammonium group, a quaternary ammonium group, and a guanidyl group

$(-NHC(=NH_2^+)NH_2)$.

<25> The polymer according to any one of <22> to <24>,

wherein the polymer further has a hydrophobic group in a side chain of the polymer,
the side chain has no disulfide linkage, and
the hydrophobic group is at least one selected from the group consisting of an aromatic cyclic group, an aliphatic cyclic group, and an aliphatic chain group.

<26> The polymer according to any one of <22> to <25>, wherein the polymer contains an amide linkage in a main chain skeleton of the polymer.
<27> The polymer according to any one of <22> to <26>,

wherein the polymer contains a structure derived from one or a plurality of amino acids in a main chain skeleton of the polymer, and
the amino acids comprise at least one selected from the group consisting of lysine, histidine, arginine, glutamic acid, aspartic acid, glutamine, asparagine, serine, and threonine.

<28> The polymer according to any one of <22> to <27>, wherein the polymer contains a biocompatible polymer as a block polymer in a main chain skeleton of the polymer.
<29> A method for producing a coated metallic base, comprising a step of bringing the polymer according to any one of <22> to <28> into contact with a surface of a metallic base to bond the hydrophilic group to the surface of the metallic base and simultaneously form a polymer layer on the surface of the metallic base.
<30> The method for producing a coated metallic base according to <29>, wherein in the side chain, a first side chain length measured from the disulfide group to the hydrophilic group is longer than a second side chain length measured from the disulfide group to a main chain of the polymer.

EXPLANATION OF REFERENCES

| | |
|---|---|
| 1 | Coated metallic base |
| 1A | Nanoparticle body |
| 1B | Coated metallic film |
| 2 | Metallic base |
| 2A | Metallic nanoparticle |
| 2B | Metallic film |
| 3 | Polymer layer |
| 3A | Polymer (constituting polymer layer) |
| 3B | Polymer (forming polymer layer) |
| 4 | Specifically bondable substance |
| 6 | Fluorescent substance |
| 7 | Hydrophilic group |
| 7A | First hydrophilic group |
| 7B | Second hydrophilic group |
| 10A | First nanoparticle body |
| 10B | Third nanoparticle body |
| 12A | First metallic nanoparticle |
| 12B | Third metallic nanoparticle |
| 13A | First polymer layer |
| 13B | Third polymer layer |
| 14A | First specifically bondable substance |
| 14B | Third specifically bondable substance |
| 16A | First fluorescent substance |
| 16B | Third fluorescent substance |
| 20A | Second nanoparticle body |
| 20B | First coated metallic base |
| 22A | Second metallic nanoparticle |

| | |
|---|---|
| 22B | First metallic film |
| 23A | Second polymer layer |
| 23B | Fourth polymer layer |
| 24A | Second specifically bondable substance |
| 24B | Fourth specifically bondable substance |
| 26A | Second fluorescent substance |
| 30 | Test substance |
| 40A, 40B | Composite |
| L | Separation distance (separative distance) |

**Claims**

**1.** A coated metallic base comprising a metallic base being a metallic nanoparticle and/or a metallic film, a polymer layer covering a surface of the metallic base, and a first hydrophilic group that inhibits nonspecific adsorption to the surface of the metallic base,

the first hydrophilic group being bonded to the surface of the metallic base, and
the polymer layer containing a binding site with a sulfur atom interposed therein between the polymer layer and the surface of the metallic base.

**2.** The coated metallic base according to claim 1, wherein the first hydrophilic group contains a binding site with a sulfur atom interposed therein between the first hydrophilic group and the surface of the metallic base.

**3.** The coated metallic base according to claim 1 or 2, wherein a polymer forming the polymer layer contains, in a side chain of the polymer, the binding site with the sulfur atom interposed therein between the side chain and the surface of the metallic base.

**4.** The coated metallic base according to any one of claims 1 to 3, wherein a polymer constituting the polymer layer contains, in a side chain of the polymer, one or a plurality of second hydrophilic groups bonded with a disulfide group interposed, per one molecular chain of the polymer.

**5.** The coated metallic base according to claim 4,

wherein the polymer further contains the second hydrophilic group in a side chain of the polymer,
the first hydrophilic group and the second hydrophilic group each independently include a polar group and/or a charged group,
the polar group includes at least one selected from the group consisting of a carboxyl group, a hydroxyl group, an amino group, a sulfonyl group, a phosphate group, and an alkylene oxide group,
the charged group includes at least one selected from the group consisting of a group in which the polar group is charged and a zwitterionic group, and the zwitterionic group includes at least one selected from the group consisting of a phosphorylcholine group and a betaine group.

**6.** The coated metallic base according to any one of claims 1 to 5, wherein a polymer constituting the polymer layer contains an amide linkage in a main chain skeleton of the polymer.

**7.** The coated metallic base according to any one of claims 1 to 6,

wherein a polymer constituting the polymer layer further contains a positively charged group in a side chain of the polymer,
the side chain has no disulfide linkage,
the positively charged group is at least one selected from the group consisting of a primary ammonium group, a secondary ammonium group, a tertiary ammonium group, a quaternary ammonium group, and a guanidyl group ($-NHC(=NH_2^+)NH_2$).

**8.** The coated metallic base according to any one of claims 1 to 7,

wherein a polymer constituting the polymer layer further contains a hydrophobic group in a side chain of the polymer,

the side chain has no disulfide linkage, and
the hydrophobic group is at least one selected from the group consisting of an aromatic cyclic group, an aliphatic cyclic group, and an aliphatic chain group.

**9.** The coated metallic base according to any one of claims 3 to 8,

wherein the polymer contains a structure derived from one or a plurality of amino acids in a main chain skeleton, and
the amino acids comprise at least one selected from the group consisting of lysine, histidine, arginine, glutamic acid, aspartic acid glutamine, asparagine, serine, and threonine.

**10.** The coated metallic base according to any one of claims 1 to 9,
wherein a polymer constituting the polymer layer contains a biocompatible polymer as a block polymer in a main chain skeleton of the polymer.

**11.** The coated metallic base according to any one of claims 1 to 10, further comprising a fluorescent substance and/or a specifically bondable substance that is to be specifically bonded to a test substance in a specimen.

**12.** The coated metallic base according to claim 11, wherein the specifically bondable substance is a nanoantibody.

**13.** The coated metallic base according to any one of claims 1 to 12, wherein the polymer layer has a thickness of 1 nm to 10 nm.

**14.** The coated metallic base according to any one of claims 1 to 13, wherein the coated metallic base is a coated metallic base to be used for plasmon-excited fluorescence analysis.

**15.** The coated metallic base according to any one of claims 1 to 14, wherein the metallic base comprises gold or silver.

**16.** The coated metallic base according to any one of claims 1 to 15,

which is a nanoparticle body including the metallic nanoparticle as the metallic base,
wherein a first nanoparticle body and a second nanoparticle body are included as the nanoparticle body,
wherein the first hydrophilic group is bonded to at least one of a surface of the metallic nanoparticle of the first nanoparticle body and a surface of the metallic nanoparticle of the second nanoparticle body, and
the coated metallic base forms a composite in which the first nanoparticle body and the second nanoparticle body are bonded to each other with a test substance interposed therebetween.

**17.** The coated metallic base according to any one of claims 1 to 16,

wherein the coated metallic base includes a nanoparticle body containing the metallic nanoparticle as the metallic base and a coated metallic film containing the metallic film as the metallic base,
the first hydrophilic group is bonded to at least one of a surface of the metallic nanoparticle and a surface of the metallic film, and
the coated metallic base forms a composite in which the coated metallic film and the nanoparticle body are bonded to each other with a test substance interposed therebetween.

**18.** The coated metallic base according to claim 16 or 17, wherein the test substance is a test substance derived from a specimen being blood, plasma, urine, or saliva.

**19.** The coated metallic base according to any one of claims 1 to 18, wherein a molecule constituting the polymer layer cover a surface of the metallic base in a two-dimensional manner such that a main chain of the polymer is substantially parallel to the surface of the metallic base.

**20.** A composite comprising two or more nanoparticle bodies containing the metallic nanoparticle according to any one of claims 1 to 19,

the two or more nanoparticle bodies include a first nanoparticle body and a second nanoparticle body, and
the first nanoparticle body and the second nanoparticle body are bonded to each other with a test substance

interposed therebetween.

**21.** A composite comprising a nanoparticle body including the metallic nanoparticle according to any one of claims 1 to 19 and a coated metallic film including the metallic film according to any one of claims 1 to 19,

wherein the first hydrophilic group is bonded to at least one of a surface of the metallic nanoparticle and a surface of the metallic film, and
the nanoparticle body and the coated metallic film are bonded to each other with a test substance interposed therebetween.

**22.** A polymer having a hydrophilic group bonded with a disulfide group interposed, at a terminal of a side chain.

**23.** The polymer according to claim 22,

wherein the hydrophilic group includes a polar group and/or a charged group,
the polar group includes at least one selected from the group consisting of a carboxyl group, a hydroxyl group, an amino group, a sulfonyl group, a phosphate group, and an alkylene oxide group, and
the charged group includes at least one selected from the group consisting of a group in which the polar group is charged and a zwitterionic group, and the zwitterionic group includes at least one selected from the group consisting of a phosphorylcholine group and a betaine group.

**24.** The polymer according to claim 22 or 23,

wherein the polymer further contains a positively charged group in a side chain of the polymer,
the side chain has no disulfide linkage, and
the positively charged group is at least one selected from the group consisting of a primary ammonium group, a secondary ammonium group, a tertiary ammonium group, a quaternary ammonium group, and a guanidyl group ($-NHC(=NH_2^+)NH_2$).

**25.** The polymer according to any one of claims 22 to 24,

wherein the polymer further contains a hydrophobic group in a side chain of the polymer,
the side chain has no disulfide linkage, and
the hydrophobic group is at least one selected from the group consisting of an aromatic cyclic group, an aliphatic cyclic group, and an aliphatic chain group.

**26.** The polymer according to any one of claims 22 to 25, wherein the polymer contains an amide linkage in a main chain skeleton of the polymer.

**27.** The polymer according to any one of claims 22 to 26,

wherein the polymer contains a structure derived from one or a plurality of amino acids in a main chain skeleton of the polymer, and
the amino acids comprise at least one selected from the group consisting of lysine, histidine, arginine, glutamic acid, aspartic acid, glutamine, asparagine, serine, and threonine.

**28.** The polymer according to any one of claims 22 to 27, wherein the polymer contains a biocompatible polymer as a block polymer in a main chain skeleton of the polymer.

**29.** A method for producing a coated metallic base, comprising a step of bringing the polymer according to any one of claims 22 to 28 into contact with a surface of a metallic base to bond the hydrophilic group to the surface of the metallic base and simultaneously form a polymer layer on the surface of the metallic base.

**30.** The method for producing a coated metallic base according to claim 29, wherein in the side chain, a first side chain length from the disulfide group to the hydrophilic group is longer than a second side chain length from the disulfide group to a main chain of the polymer.

*Fig.1*

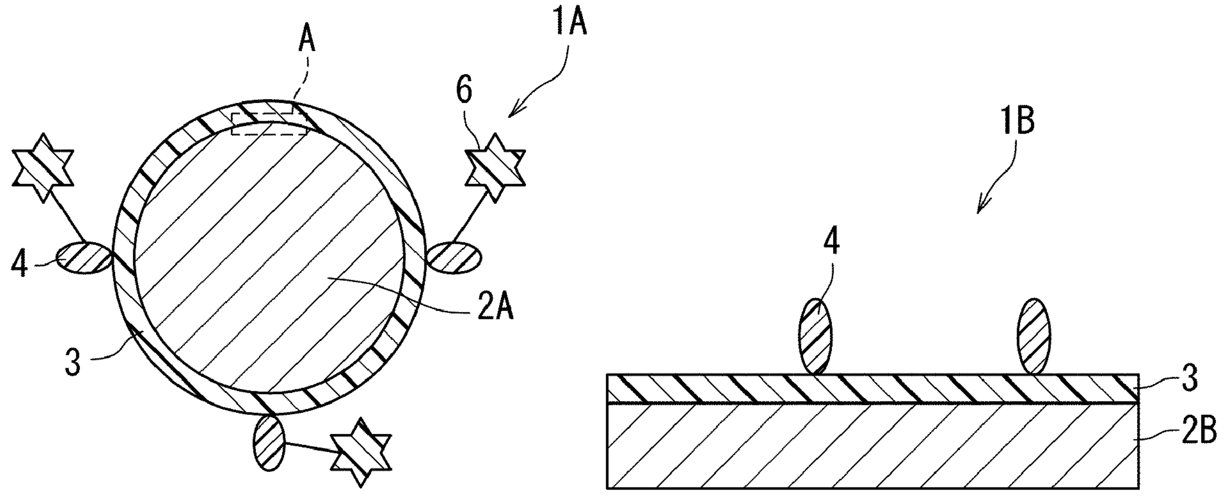


(a) (b)

*Fig.2*

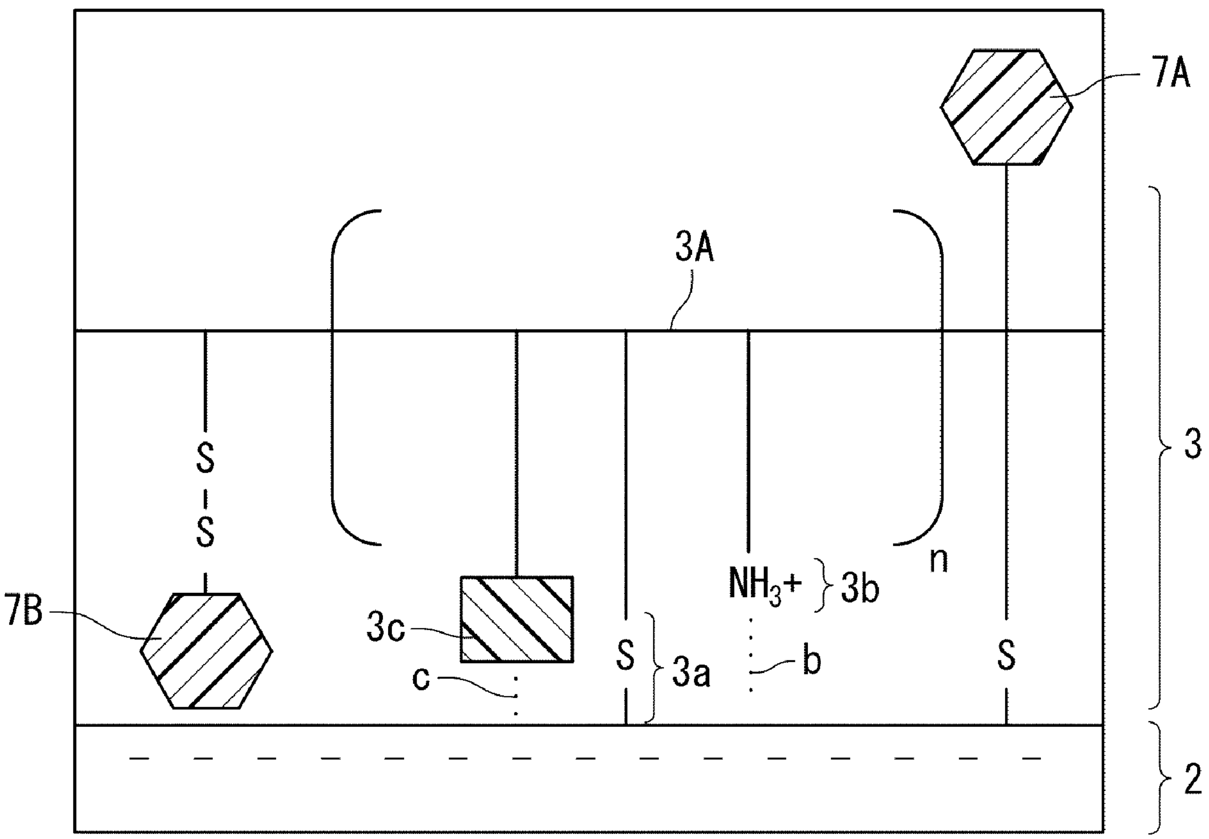

*Fig.3*

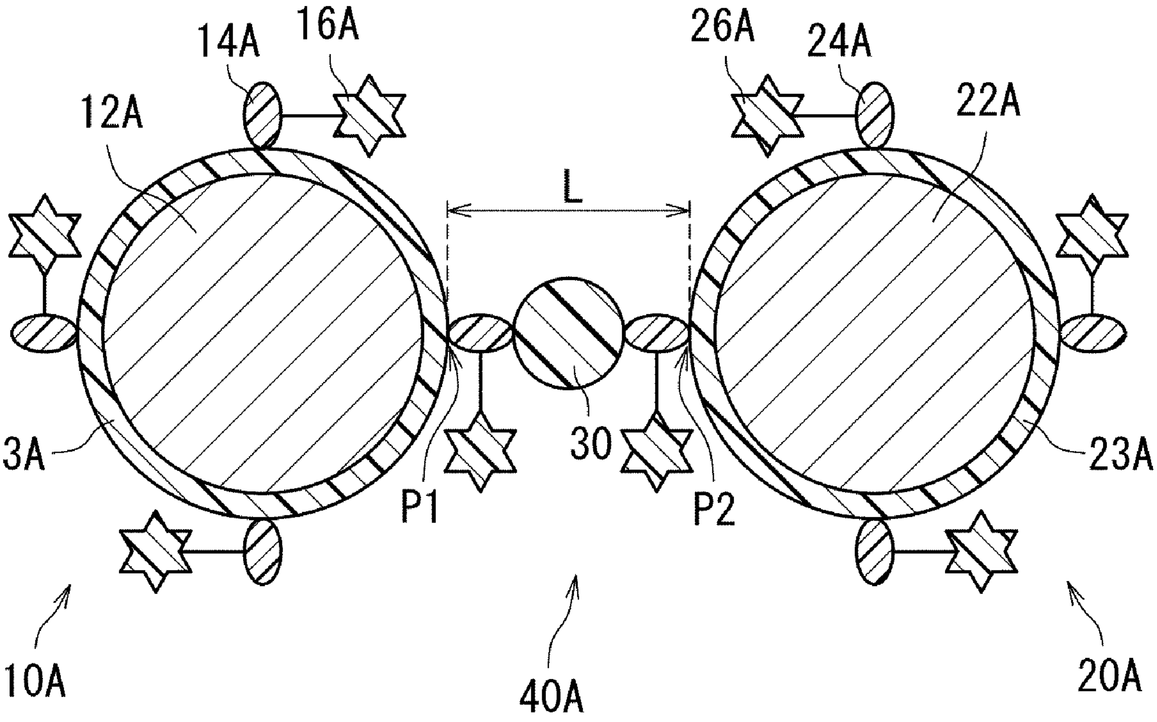
14A
16A
26A
24A
12A
22A
L
13A
30
23A
P1
P2
10A
40A
20A

*Fig.4*

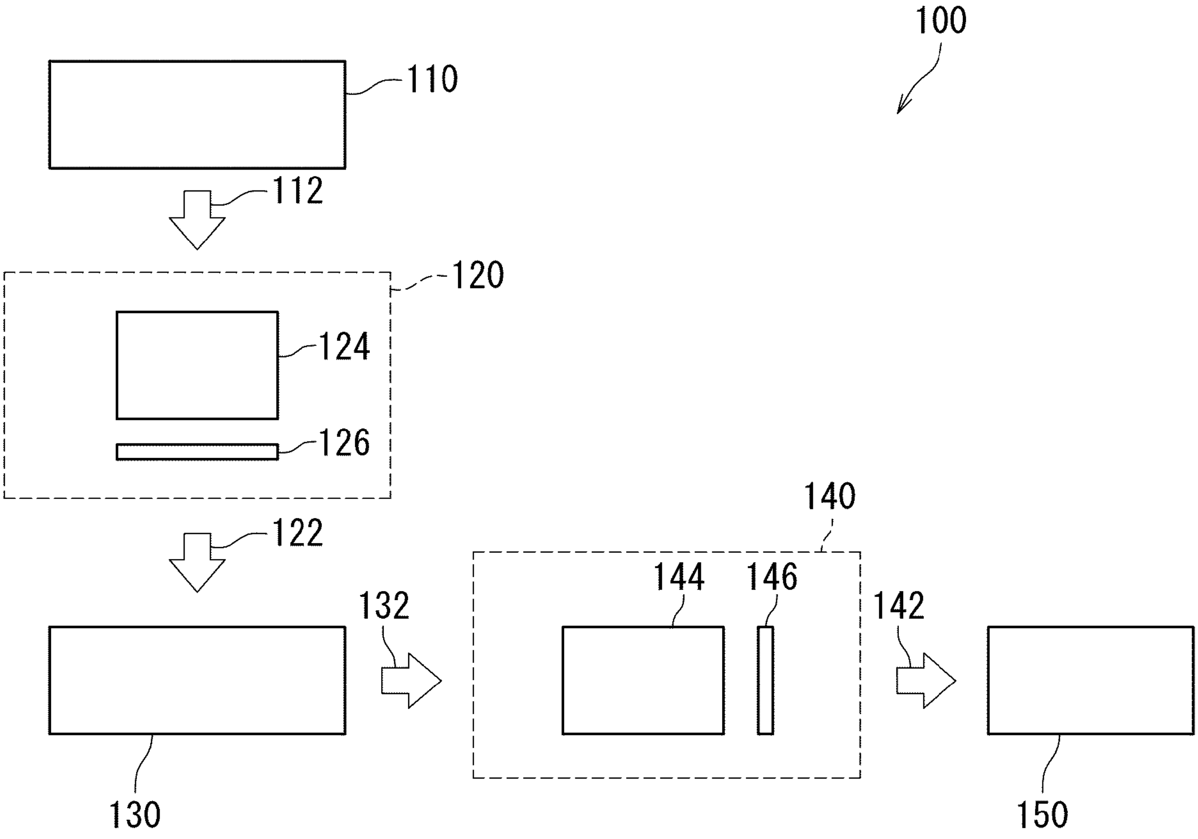
100
110
112
120
124
126
122
140
144
146
132
142
130
150

Fig.5

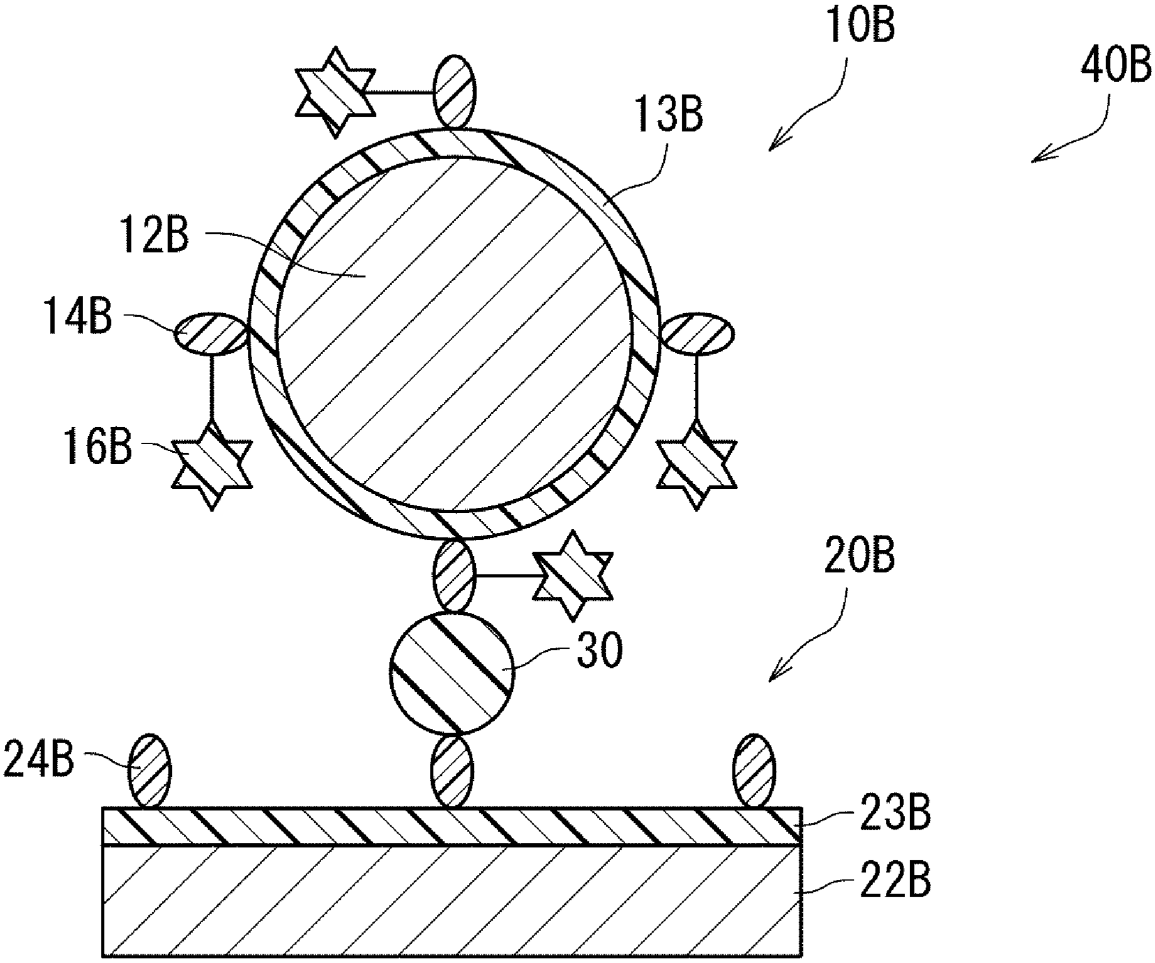
10B
40B
13B
12B
14B
16B
20B
30
24B
23B
22B

Fig.6

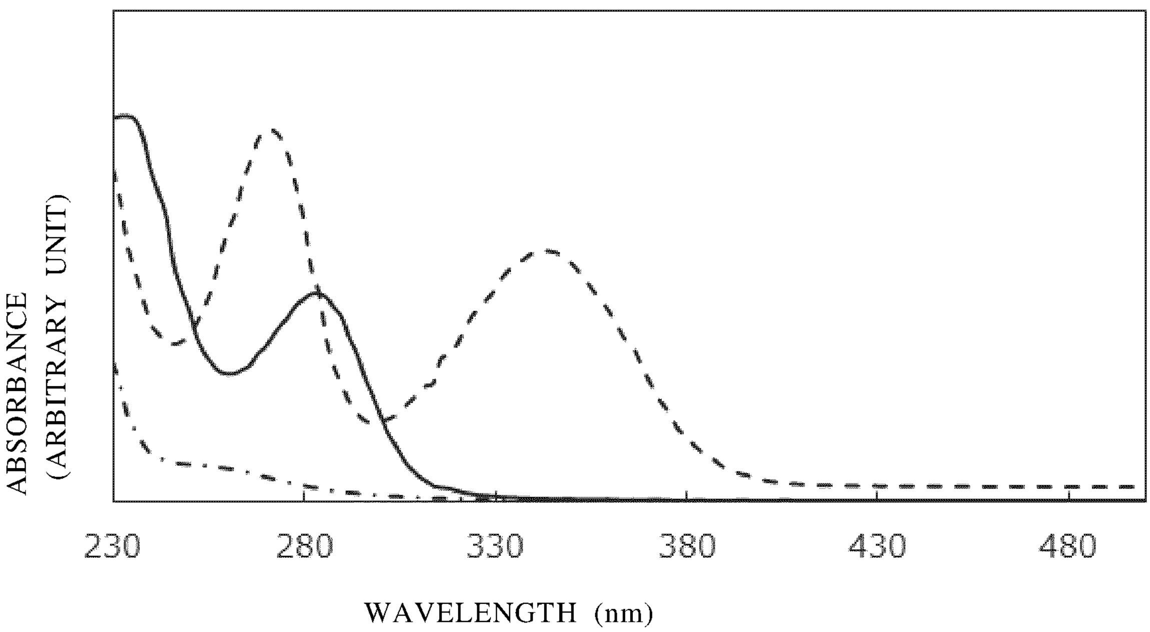
ABSORBANCE
(ARBITRARY UNIT)
230
280
330
380
430
480
WAVELENGTH (nm)

*Fig.7*

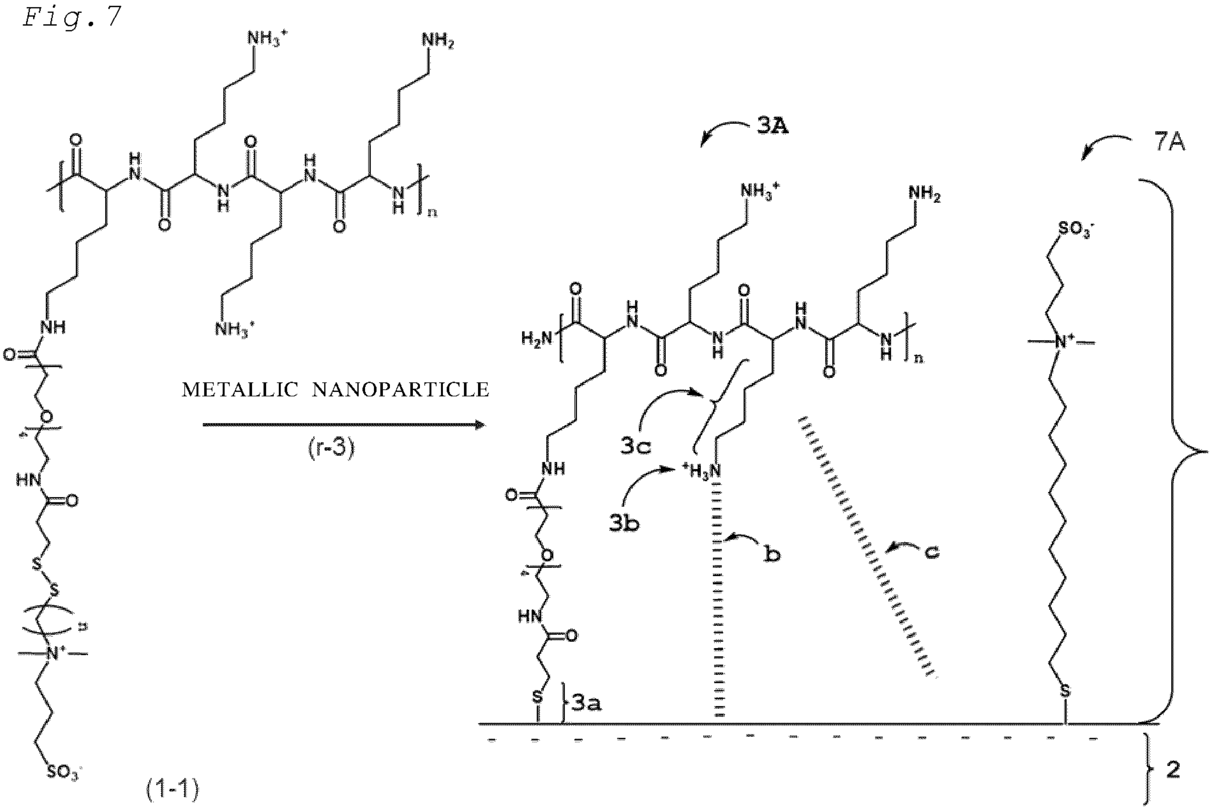


(1-1)

Fig.8

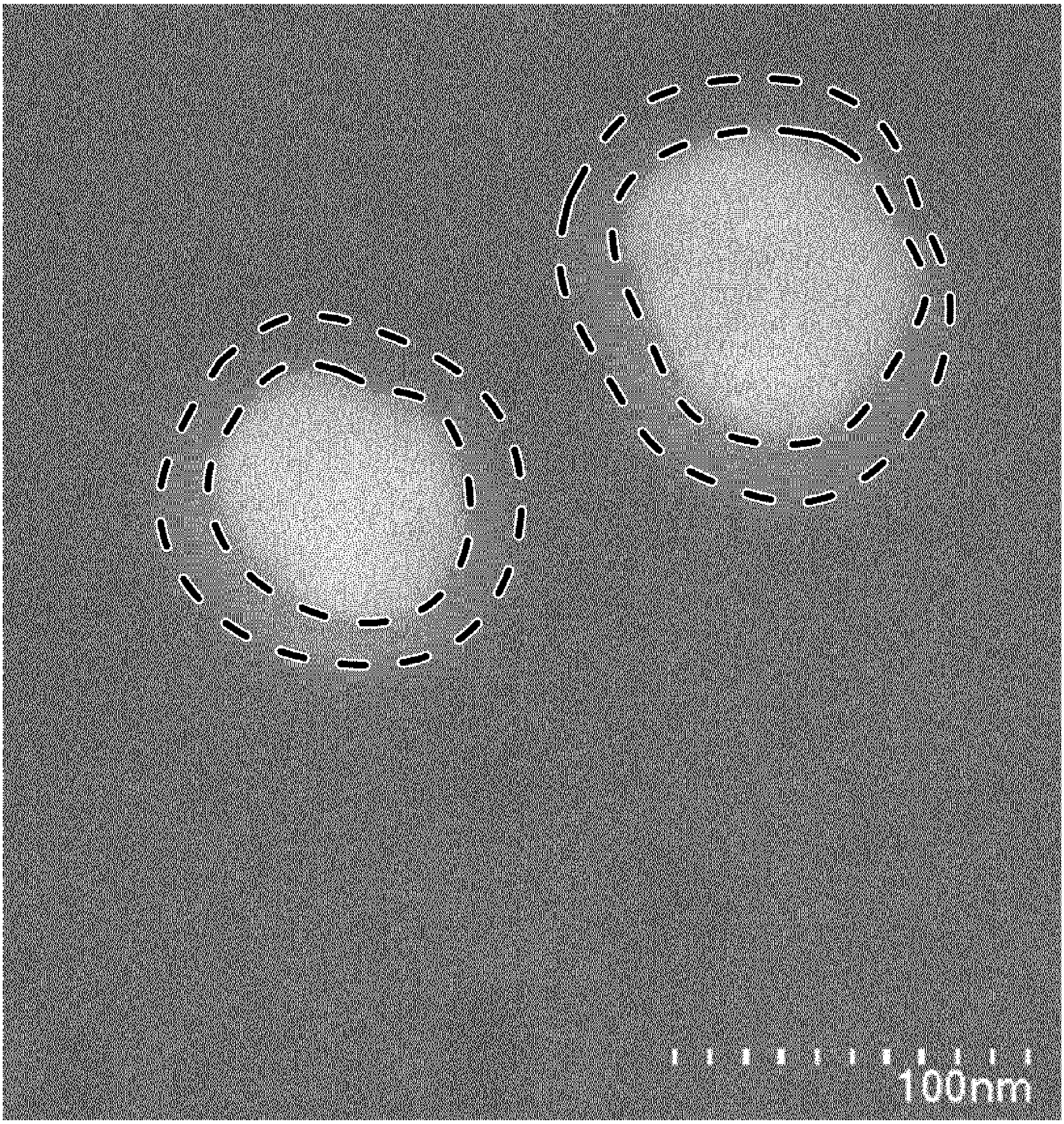

*Fig. 9*

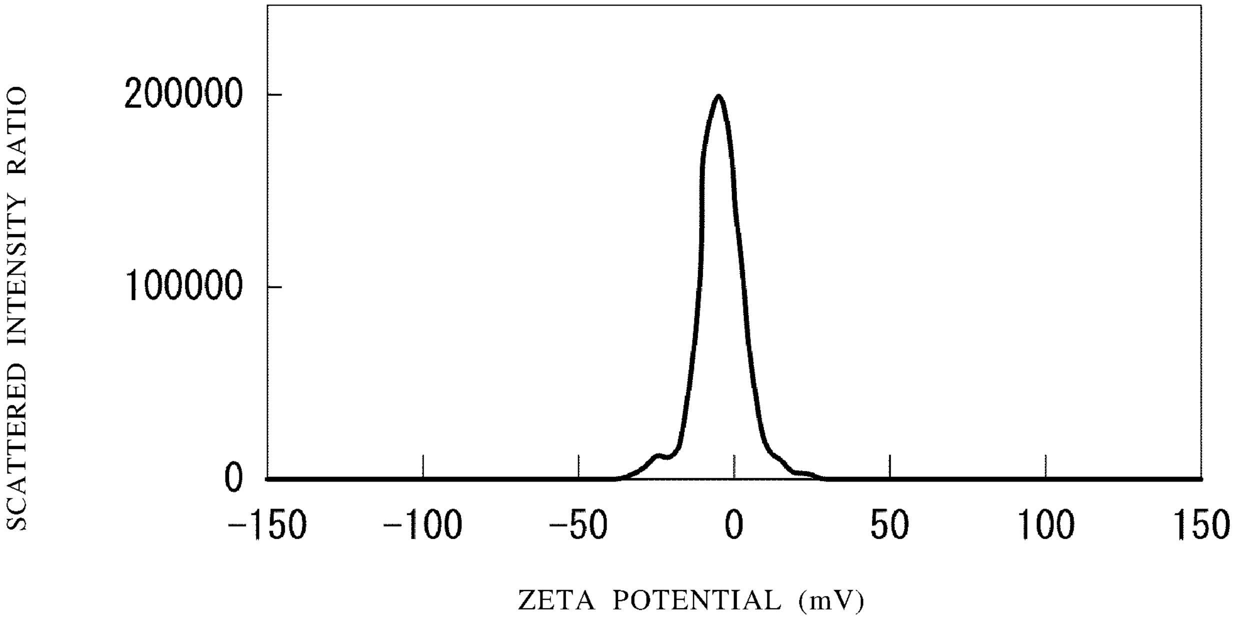
SCATTERED INTENSITY RATIO
200000
100000
0
-150
-100
-50
0
50
100
150
ZETA POTENTIAL (mV)

*Fig. 10*

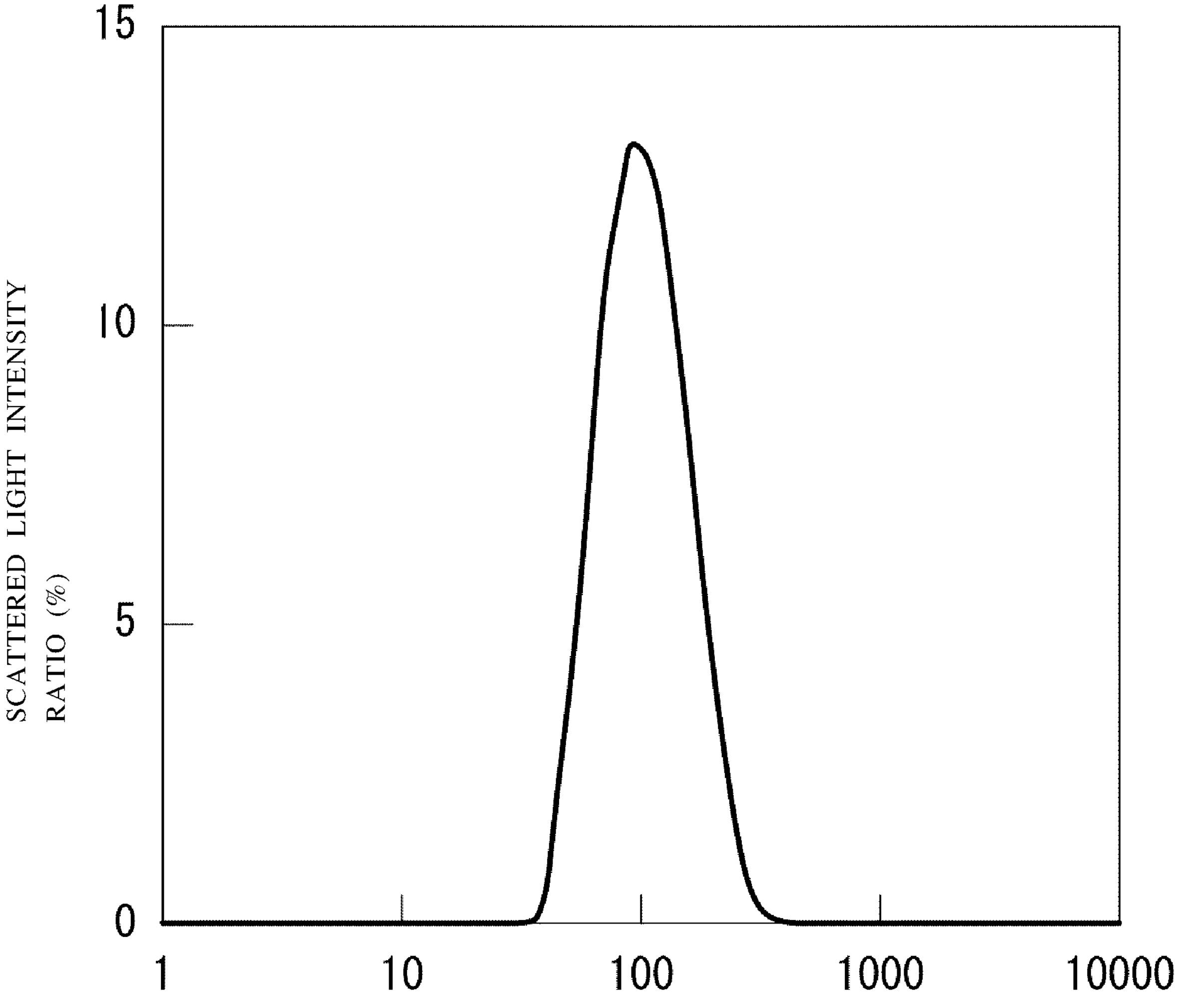
SCATTERED LIGHT INTENSITY RATIO (%)
15
10
5
0
1
10
100
1000
10000
PARTICLE DIAMETER (nm)

*Fig.11*

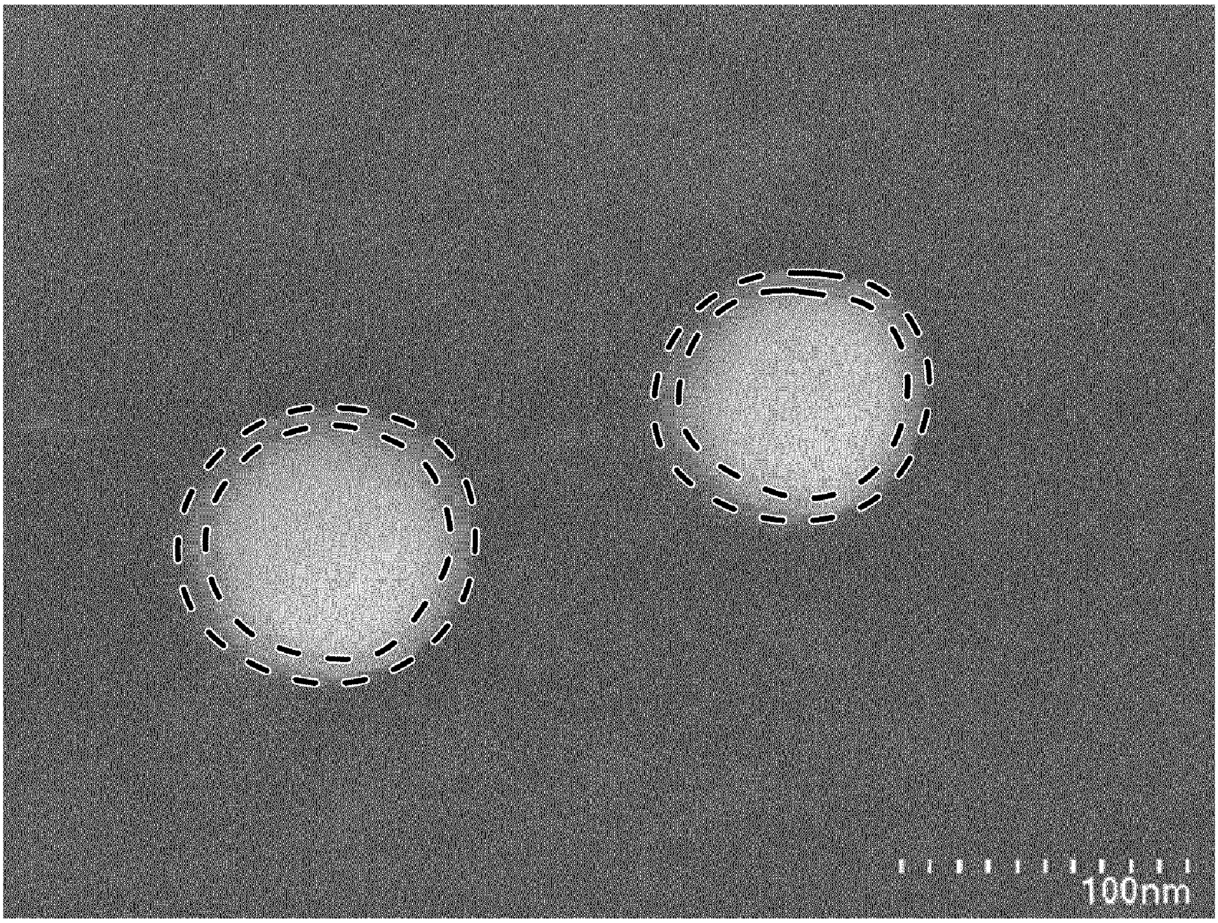
100nm

*Fig.12*

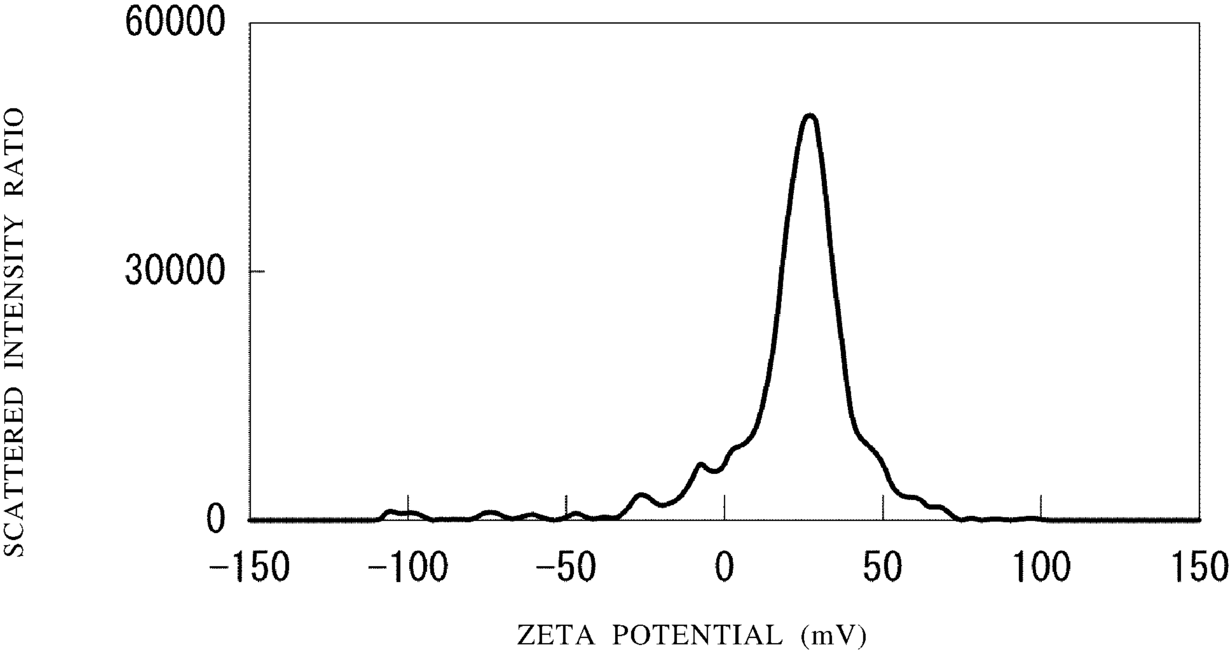
60000
30000
0
SCATTERED INTENSITY RATIO
−150
−100
−50
0
50
100
150
ZETA POTENTIAL (mV)

**INTERNATIONAL SEARCH REPORT**

International application No. **PCT/JP2023/018462**

| | |
|---|---|
| **A. CLASSIFICATION OF SUBJECT MATTER** | |
| ***G01N 33/553***(2006.01)i; ***C08G 75/0268***(2016.01)i; ***G01N 21/64***(2006.01)i; ***G01N 33/543***(2006.01)i<br>FI: G01N33/553; G01N33/543 525W; G01N33/543 525U; G01N33/543 501J; G01N33/543 595; G01N33/543 575; G01N21/64 Z; C08G75/0268 | |
| According to International Patent Classification (IPC) or to both national classification and IPC | |
| **B. FIELDS SEARCHED** | |
| Minimum documentation searched (classification system followed by classification symbols)<br>G01N33/553; C08G75/0268; G01N21/64; G01N33/543 | |
| Documentation searched other than minimum documentation to the extent that such documents are included in the fields searched<br>Published examined utility model applications of Japan 1922-1996<br>Published unexamined utility model applications of Japan 1971-2023<br>Registered utility model specifications of Japan 1996-2023<br>Published registered utility model applications of Japan 1994-2023 | |
| Electronic data base consulted during the international search (name of data base and, where practicable, search terms used)<br>JSTPlus/JMEDPlus/JST7580 (JDreamIII); CAplus/MEDLINE/EMBASE/BIOSIS (STN) | |

**C. DOCUMENTS CONSIDERED TO BE RELEVANT**

| Category* | Citation of document, with indication, where appropriate, of the relevant passages | Relevant to claim No. |
|---|---|---|
| X | JP 2009-292804 A (CANON INC) 17 December 2009 (2009-12-17)<br>paragraphs [0015]-[0082], fig. 1-13 | 1, 3, 8, 11-13, 15 |
| X | JP 2009-145189 A (FUJIFILM CORP) 02 July 2009 (2009-07-02)<br>paragraphs [0012]-[0088], fig. 1-2 | 1-3, 11-15 |
| A | JP 2009-300239 A (CANON INC) 24 December 2009 (2009-12-24)<br>entire text, all drawings | 1-30 |
| A | JP 2016-057106 A (JSR CORP) 21 April 2016 (2016-04-21)<br>entire text, all drawings | 1-30 |
| P, A | WO 2022/250055 A1 (PHC HOLDINGS CORP) 01 December 2022 (2022-12-01)<br>entire text, all drawings | 1-30 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **31 July 2023** | **08 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/018462**

**Box No. III Observations where unity of invention is lacking (Continuation of item 3 of first sheet)**

This International Searching Authority found multiple inventions in this international application, as follows:

Claims 1 to 30 in the present application are considered to consist of the following two invention groups.

(Invention 1)
Claims 1-21.

(Invention 2)
Claims 22-30.

Claim 22, which is an independent claim and sets forth an invention of a “polymer having, at the end of a side chain, a hydrophilic group bonded via a disulfide group”, shares the common technical feature of the “hydrophilic group” and the “polymer” with claim 1. However, said common feature is well known to those skilled in the art without needing to provide any example and thus cannot be considered a special technical feature.

Also, claims 22-30 are not dependent on claim 1. In addition, claims 22-30 are not substantially identical to or similarly closely related to any of the claims classified as invention 1.

Therefore, claims 22-30 are classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant’s protest and, where applicable, the payment of a protest fee.

☐ The additional search fees were accompanied by the applicant’s protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/018462**

| Patent document cited in search report | Publication date (day/month/year) | Patent family member(s) | Publication date (day/month/year) |
|---|---|---|---|
| JP 2009-292804 A | 17 December 2009 | US 2009/0137064 A1<br>paragraphs [0026]-[0118], fig. 1-13 | |
| JP 2009-145189 A | 02 July 2009 | US 2009/0153869 A1<br>paragraphs [0018]-[0129], fig. 1-2<br>EP 2071321 A2 | |
| JP 2009-300239 A | 24 December 2009 | US 2010/0330704 A1<br>entire text, all drawings<br>WO 2009/151148 A1 | |
| JP 2016-057106 A | 21 April 2016 | US 2016/0069871 A1<br>entire text, all drawings | |
| WO 2022/250055 A1 | 01 December 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001086301 A